# EUROPEAN PATENT APPLICATION

(11) **EP 4 151 206 A1**
(43) Date of publication of application: **22.03.2023**
(21) Application number: 21804321.4
(22) Date of filing: 13.05.2021
(51) Int. Cl.: A61K 31/02, A61K 33/00, A61M 31/00, A61P 7/00, A61P 11/00

(54) **PHARMACEUTICAL COMPOSITION USED TO TREAT SUBJECT IN HYPOXIC STATE DUE TO RESPIRATORY FAILURE, ETC**

(30) Priority: 13.05.2020 JP 2020084395
(71) Applicant: National University Corporation Tokyo Medical and Dental University, Tokyo 113-8510 (JP)
(72) Inventor: TAKEBE Takanori, Tokyo 113-8510 (JP); YONEYAMA Yosuke, Tokyo 113-8510 (JP); OKABE Ryo, Kyoto-shi Kyoto 606-8501 (JP); YOSHIKAWA Toyofumi, Kyoto-shi Kyoto 606-8501 (JP); DATE Hiroshi, Kyoto-shi Kyoto 606-8501 (JP)
(74) Representative: Marks & Clerk LLP
(86) International application number: PCT/JP2021/018213
(87) International publication number: WO 2021/230317

(57) **Abstract**

[Abstract] The present invention provides a pharmaceutical composition for use in administering oxygen to a subject, the pharmaceutical composition containing a perfluorocarbon dissolving oxygen therein. The present invention also provides a pharmaceutical composition for use in decreasing the blood carbon dioxide partial pressure of a subject, the pharmaceutical composition containing a perfluorocarbon.

## Description

### Technical Field

The present invention relates to a pharmaceutical composition for use in treating a subject in a hypoxic state due to respiratory failure, etc.

### Background Art

Artificial respiratory assistance is playing an important role in clinical management of respiratory failure due to severe diseases including pneumonia and acute respiratory distress syndrome. Mechanical artificial ventilation is the most commonly used short-term life support technique in the world^{1,2}. Oxygenation, also called extracorporeal membrane oxygenation (ECMO), is an approach alternative to ventilators for patients having severe respiratory failure who are intolerable to mechanical ventilation, and generally used as a temporary palliative therapy³. However, the recent SARS-CoV-2 pandemic overwhelms clinical needs of ventilators and oxygenation, causing critical shortage of available devices, and threatening the lives of patients around the world. In a small-scale study by Yang et al., comparison of clinical features and outcomes across different treatment methods for patients having severe SARS-CoV-2 showed that five of six patients (83%) subjected to ECMO died^{4,5}. Moreover, ECMO requires huge manpower and enormous healthcare cost. Other complexes due to ECMO include neurological complexes such as seizure, ischemic stroke, intracranial hemorrhage, and brain death, and hemorrhage is the most common complex in patients under ECMO³. Currently, there is no other effective indwelling respiratory device, and hence it is needed to develop supportive therapy for severe respiratory failure.

Amelioration of a hypoxic state is the most critical factor for amelioration of respiratory failure. Molecular oxygen is a major substrate essential for mitochondrial ATP production and numerous intracellular biochemical reactions for most organisms⁶. Notably, not only some unique non-mammals but also some mammalian species have evolved to survive in and adapt to hypoxic environments. These species need an accessory respiratory mechanism in an organ other than lungs and gills. For example, subspecies of frogs and toads use the skin for respiration, loaches (*Misgumus anguillicandatus*), sea cucumbers, corydorases, and *Tetragnatha praedonia* use the intestine for respiration, and naked mole rats use fructose-driven glycolysis⁷⁻¹⁰. Interestingly, loaches normally perform bronchial respiration in standard oxygen environments, and in hypoxic environments switch the posterior part of the intestine to an auxiliary respiratory site for survival. Loaches and other species living in a hypoxic environment for a long period of time enhance their antioxidative systems and immunological defenses¹¹, and simultaneously convert their digestion functions to fit to intestinal respiration by changing transporter and vascularization genes.

### Summary of Invention

The present invention provides a pharmaceutical composition for use in treating a subject in a hypoxic state due to respiratory failure, etc.

The present inventors revealed that the blood oxygen partial pressure of a subject can be improved by allowing the intestinal tract (the intestinal tract preferably in the large intestine, more preferably in the rectum) subjected to mucosal abrasion to absorb oxygen gas; and that the blood oxygen partial pressure of a subject can be improved by administering a perfluorocarbon solution dissolving oxygen therein to the intestinal tract (preferably into the large intestine, more preferably into the rectum), even without abrasing the mucosa of the intestinal tract of the subject. This method was applied to model animals with moderate to severe acute respiratory distress syndrome (ARDS) to succeed in improving their arterial blood oxygen saturation and venous oxygen partial pressure. Furthermore, the present inventors revealed that decrease in the blood carbon dioxide partial pressure of a subject can be induced by administering a perfluorocarbon solution to the intestinal tract (preferably into the large intestine, more preferably into the rectum). Intrarectal administration of a perfluorocarbon dissolving oxygen therein successfully increased the blood oxygen partial pressure of a subject and decreased the blood carbon dioxide partial pressure.

The present invention provides the followings.
(1) A pharmaceutical composition for intraintestinal administration, the pharmaceutical composition containing a perfluorocarbon dissolving oxygen therein.
(2) The pharmaceutical composition according to (1), for use in treating hypoxemia.
(3) The pharmaceutical composition according to (1) or (2), wherein the pharmaceutical composition is administered to a subject having respiratory failure.
(4) The pharmaceutical composition according to any one of (1) to (3), for use in supplying oxygen to the blood of a subject.
(5) An administration device for intraintestinal administration, the administration device including the pharmaceutical composition according to any one of (1) to (4) .
(6) A pharmaceutical composition in the form of gas, comprising oxygen gas, for intraintestinal administration.
(7) The pharmaceutical composition according to (6), wherein the intestinal tract is an intestinal tract subjected to mucosal removal or an intestinal tract at least partially coated with a perfluorocarbon.
(8) An administration device for intraintestinal administration, the administration device including the pharmaceutical composition according to (6) or (7).
(9) An administration controller for administering a perfluorocarbon dissolving oxygen therein, the administration controller including: a delivering unit configured to deliver the perfluorocarbon dissolving oxygen therein or oxygen to a tube; and a controlling unit configured to control the rate of delivery from the delivering unit on the basis of the blood oxygen saturation of a subject and/or the intraintestinal pressure of a subject.
(10) The administration controller according to (9), further including a receiving unit configured to receive information on oxygen saturation from a blood oxygen monitor and/or intraintestinal pressure.
(11) The administration controller according to (9) or (10), including:
   a controlling unit configured to control the rate of delivery from the delivering unit on the basis of the blood oxygen saturation of the subject, wherein
   (A) the controlling unit refers to information on oxygen saturation received by a receiving unit, and if the oxygen saturation has reached lower than a predetermined value or a predetermined value or lower, the controlling unit sends a signal to increase the rate of delivery to the delivering unit; and/or
   (B) the controlling unit refers to information on oxygen saturation received by a receiving unit, and if the oxygen saturation has reached a predetermined value or higher or higher than a predetermined value, the controlling unit sends a signal to decrease the rate of delivery to the delivering unit.
(12) The administration controller according to any one of (9) to (11), including:
   a controlling unit configured to control the rate of delivery from the delivering unit on the basis of the intraintestinal pressure of the subject, wherein
   (C) the controlling unit refers to information on intraintestinal pressure received by a receiving unit, and if the intraintestinal pressure has reached lower than a predetermined value or a predetermined value or lower, the controlling unit stops sending a signal to increase the rate of delivery to the delivering unit, or sends a signal to decrease the rate of delivery to the delivering unit.
(13) An administration controller for administering oxygen gas, the administration controller including: an gas-delivering unit configured to deliver a perfluorocarbon dissolving oxygen therein or oxygen to a tube; and a controlling unit configured to control the rate of gas delivery from the gas-delivering unit on the basis of the blood oxygen saturation and/or intraintestinal pressure of a subject.
(14) The administration controller according to (13), further including a receiving unit configured to receive information on oxygen saturation from a blood oxygen monitor and/or intraintestinal pressure.
(15) The administration controller according to (13) or (14), including:
   a controlling unit configured to control the rate of gas delivery from the gas-delivering unit on the basis of the blood oxygen saturation of a subject, wherein
   (A) the controlling unit refers to information on oxygen saturation received by a receiving unit, and if the oxygen saturation has reached lower than a predetermined value or a predetermined value or lower, the controlling unit sends a signal to increase the rate of gas delivery to the gas-delivering unit; and/or
   (B) the controlling unit refers to information on oxygen saturation received by a receiving unit, and if the oxygen saturation has reached a predetermined value or higher or higher than a predetermined value, the controlling unit sends a signal to decrease the rate of gas delivery to the gas-delivering unit.
(16) The administration controller according to any one of (13) to (15), including:
   a controlling unit configured to control the rate of gas delivery from the gas-delivering unit on the basis of the intraintestinal pressure of a subject, wherein
   (C) the controlling unit refers to information on intraintestinal pressure received by a receiving unit, and if the intraintestinal pressure has reached lower than a predetermined value or a predetermined value or lower, the controlling unit stops sending a signal to increase the rate of gas delivery to the gas-delivering unit, or sends a signal to decrease the rate of gas delivery to the gas-delivering unit.

The present invention provides the followings.
(1A) A pharmaceutical composition for oral administration, trans-fistula gastric administration, nasogastric administration, or administration into a large intestine, the pharmaceutical composition containing a perfluorocarbon dissolving oxygen therein.
(2A) The pharmaceutical composition according to (1A), for use in treating hypoxemia.
(3A) The pharmaceutical composition according to (1A) or (2A), wherein the pharmaceutical composition is administered to a subject having respiratory failure.
(4A) The pharmaceutical composition according to any one of (1A) to (3A), for use in supplying oxygen to the blood of a subject.
(5A) An administration device for administration into a large intestine, the administration device including the pharmaceutical composition according to any one of (1A) to (4A).
(6A) A pharmaceutical composition in the form of gas, comprising oxygen gas, for oral administration, nasogastric administration, trans-fistula gastric administration, or administration into a large intestine, wherein
   the large intestine is a large intestine subjected to mucosal removal or a large intestine coated with a perfluorocarbon.
(7A) A composition in the form of gas, comprising oxygen gas, wherein the composition is dissolved in a perfluorocarbon before administration and administered through oral administration, trans-fistula gastric administration, or administration into a large intestine.
(8A) The composition according to any one of claims 1 to 4, wherein the composition is mixed with oxygen gas before administration and administered through oral administration, nasogastric administration, trans-fistula gastric administration, or administration into a large intestine.
(9A) A composition in the form of gas, comprising oxygen gas, for use in administration into a large intestine to a subject having a large intestine subjected to mucosal removal or a large intestine coated with a perfluorocarbon.
(10A) The composition according to any one of (1A) to (4A) and (6A) to (9A) for intrarectal administration.
(11A) The composition according to any one of (1A) to (4A) and (6A) to (9A) for oral administration, nasogastric administration, or trans-fistula gastric administration.
(12A) The composition according to any one of (1A) to (4A) and (6A) to (11A), for use in decreasing the blood carbon dioxide partial pressure of a subject.
(13A) A kit for pre-use preparation of a composition for administration into a large intestine, the kit including: a composition in the form of gas, containing oxygen gas; and a composition containing a perfluorocarbon.
(14A) The kit for pre-use preparation according to (13A), for use in increasing the blood oxygen partial pressure of a subject.
(15A) The kit for pre-use preparation according to (13A) or (14A), for use in decreasing the blood carbon dioxide partial pressure of a subject.
(16A) An administration device for administration into a large intestine, the administration device including the composition according to any one of (1A) to (4A) and (6A) to (12A).
(17A) An administration controller for oral administration,, trans-fistula gastric administration, nasogastric administration, or administration into a large intestine of a perfluorocarbon dissolving oxygen therein or oxygen, the administration controller including: a delivering unit configured to deliver the perfluorocarbon dissolving oxygen therein or oxygen to a tube; and a controlling unit configured to control the rate of delivery from the delivering unit on the basis of the blood oxygen saturation of a subject and the intraintestinal pressure of the large intestine of the subject.
(18A) The administration controller according to (17A), further including a receiving unit configured to receive information on oxygen saturation from a blood oxygen monitor and intraintestinal pressure of the large intestine.
(19A) The administration controller according to (17A) or (18A), including:
   a controlling unit configured to control the rate of delivery from the delivering unit on the basis of the blood oxygen saturation of the subject, wherein
   (A) the controlling unit refers to information on oxygen saturation received by a receiving unit, and if the oxygen saturation has reached lower than a predetermined value or a predetermined value or lower, the controlling unit sends a signal to increase the rate of delivery to the delivering unit; and/or
   (B) the controlling unit refers to information on oxygen saturation received by a receiving unit, and if the oxygen saturation has reached a predetermined value or higher or higher than a predetermined value, the controlling unit sends a signal to decrease the rate of delivery to the delivering unit.
(20A) The administration controller according to any one of (17A) to (19A), further including:
   a controlling unit configured to control the rate of delivery from the delivering unit on the basis of the intraintestinal pressure of the large intestine of the subject, wherein
   (C) the controlling unit refers to information on intraintestinal pressure of the large intestine received by a receiving unit, and if the intraintestinal pressure has reached lower than a predetermined value or a predetermined value or lower or if the intraintestinal pressure has increased during liquid delivery, the controlling unit stops sending a signal to increase the rate of delivery to the delivering unit, or sends a signal to decrease the rate of delivery to the delivering unit.
(21A) A method for administering oxygen to a subject, the method including:
   administering a pharmaceutical composition containing a perfluorocarbon dissolving oxygen therein to the subject through oral administration, nasogastric administration, or administration into a large intestine.
(22A) The method according to (21A), including:
   administering a perfluorocarbon dissolving oxygen therein to the subject through oral administration, nasogastric administration, trans-fistula gastric administration, or administration into a large intestine with dose control using an administration controller, wherein
   the administration controller is an administration controller for administering a perfluorocarbon dissolving oxygen therein or oxygen, and includes: a delivering unit configured to deliver the perfluorocarbon dissolving oxygen therein or oxygen to a tube; and a controlling unit configured to control the rate of delivery from the delivering unit on the basis of the blood oxygen saturation of the subject and/or the intraintestinal pressure of the large intestine of the subject.
(23A) The method according to (22A) or (23A), wherein the large intestine is a rectum.
(24A) The composition according to any one of (1A) to (4A) and (6A) to (12A), for administering a perfluorocarbon dissolving oxygen therein to a subject through oral administration, nasogastric administration, trans-fistula gastric administration, or administration into a large intestine with dose control using an administration controller, wherein
   the administration controller is an administration controller for administering a perfluorocarbon dissolving oxygen therein or oxygen, and includes: a delivering unit configured to deliver the perfluorocarbon dissolving oxygen therein or oxygen to a tube; and a controlling unit configured to control the rate of delivery from the delivering unit on the basis of the blood oxygen saturation of the subject and/or the intraintestinal pressure of the large intestine of the subject.
(25A) An administration controller for administering oxygen gas to a large intestine (preferably a rectum), the administration controller including: a gas-delivering unit configured to deliver oxygen to a tube; and a controlling unit configured to control the rate of gas delivery from the gas-delivering unit on the basis of the blood oxygen saturation of a subject and/or the intraintestinal pressure of the large intestine (preferably the rectum) of a subject.
(26A) The administration controller according to (25A), further including a receiving unit configured to receive information on oxygen saturation from a blood oxygen monitor and/or intraintestinal pressure of the large intestine (preferably the rectum).
(27A) The administration controller according to (25A) or (26A), including:
   a controlling unit configured to control the rate of gas delivery from the gas-delivering unit on the basis of the blood oxygen saturation of the subject, wherein
   (A) the controlling unit refers to information on oxygen saturation received by a receiving unit, and if the oxygen saturation has reached lower than a predetermined value or a predetermined value or lower, the controlling unit sends a signal to increase the rate of gas delivery to the gas-delivering unit; and/or
   (B) the controlling unit refers to information on oxygen saturation received by a receiving unit, and if the oxygen saturation has reached a predetermined value or higher or higher than a predetermined value, the controlling unit sends a signal to decrease the rate of gas delivery to the gas-delivering unit.
(28A) The administration controller according to any one of (25A) to (27A) including:
   a controlling unit configured to control the rate of gas delivery from the gas-delivering unit on the basis of the intraintestinal pressure of the large intestine (preferably the rectum) of the subject, wherein
   (C) the controlling unit refers to information on intraintestinal pressure of the large intestine (preferably the rectum) received by a receiving unit, and if the intraintestinal pressure has reached lower than a predetermined value or a predetermined value or lower, the controlling unit stops sending a signal to increase the rate of gas delivery to the gas-delivering unit, or sends a signal to decrease the rate of gas delivery to the gas-delivering unit.
(29A) A pharmaceutical composition containing a perfluorocarbon dissolving oxygen therein, wherein the pharmaceutical composition is administered to a human through oral administration.
(30A) A pharmaceutical composition containing a perfluorocarbon dissolving oxygen therein, wherein the pharmaceutical composition is administered to a human through nasogastric administration.
(31A) A pharmaceutical composition containing a perfluorocarbon dissolving oxygen therein, wherein the pharmaceutical composition is administered to a human through trans-fistula gastric administration.
(32A) A pharmaceutical composition containing a perfluorocarbon dissolving oxygen therein, wherein the pharmaceutical composition is administered to a human through administration into a large intestine.
(33A) The pharmaceutical composition according to any one of (29A) to (32A), wherein the perfluorocarbon has an oxygen saturation of 50% or higher, preferably of 60% or higher, more preferably of 70% or higher, further preferably of 80% or higher, furthermore preferably of 90% or higher, especially preferably of 95% or higher.
(34A) The pharmaceutical composition according to any one of (29A) to (33A), wherein the administration is administration with an administration device.
(35A) The pharmaceutical composition according to any one of (29A) to (34A), wherein the perfluorocarbon is a perfluorocarbon maintained in the atmosphere.
(36A) The pharmaceutical composition according to any one of (29A) to (34A), wherein the perfluorocarbon has a dissolved oxygen content higher than that in the atmosphere; for example, the perfluorocarbon can be used after further dissolving oxygen therein.
(37A) The pharmaceutical composition according to any one of (29A) to (36A), wherein the human has hypoxemia.
(38A) The pharmaceutical composition according to any one of (29A) to (37A), wherein the human has a blood carbon dioxide partial pressure higher than 45 Torr.
(39A) The pharmaceutical composition according to any one of (29A) to (38A), wherein the human has respiratory failure.
(40A) The pharmaceutical composition according to (39A), wherein the human has pneumonia associated with respiratory failure.
(41A) The pharmaceutical composition according to any one of (29A) to (39A), wherein the human has asthma.
(42A) The pharmaceutical composition according to any one of (29A) to (39A), wherein the human has chronic obstructive pulmonary disease (COPD).
(43A) The pharmaceutical composition according to any one of (29A) to (39A), wherein the human has any disease listed in Table 1.

The present invention provides the followings.
(1B) A pharmaceutical composition for oral administration, nasogastric administration, or intrarectal administration, the pharmaceutical composition containing a perfluorocarbon dissolving oxygen therein.
(2B) The pharmaceutical composition according to (1B), for use in treating hypoxemia.
(3B) The pharmaceutical composition according to (1B) or (2B), wherein the pharmaceutical composition is administered to a subject having respiratory failure.
(4B) The pharmaceutical composition according to any one of (1B) to (3B), for use in supplying oxygen to the blood of a subject.
(5B) An administration device for intrarectal administration, the administration device including the pharmaceutical composition according to any one of (1B) to (4B).
(6B) A pharmaceutical composition in the form of gas, containing oxygen gas, for oral administration, nasogastric administration, or intrarectal administration, wherein
   the rectum is a rectum subjected to mucosal removal or a rectum coated with a perfluorocarbon.
(7B) A composition in the form of gas, containing oxygen gas, wherein the composition is dissolved in a perfluorocarbon before administration and administered through oral administration or intrarectal administration.
(8B) The composition according to any one of (1B) to (4B), wherein the composition is mixed with oxygen gas before administration and administered through oral administration, nasogastric administration, trans-fistula gastric administration, or administration into a large intestine.
(9B) A composition in the form of gas, containing oxygen gas, for use in intrarectal administration to a subject having a rectum subjected to mucosal removal or a rectum coated with a perfluorocarbon.
(10B) The composition according to any one of (1B) to (4B) and (6B) to (9B) for intrarectal administration.
(11B) The composition according to any one of (1B) to (4B) and (6B) to (9B) for oral administration or nasogastric administration.
(12B) The composition according to any one of (1B) to (4B) and (6B) to (11B), for use in decreasing the blood carbon dioxide partial pressure of the subject.
(13B) A kit for pre-use preparation of a composition for intrarectal administration, the kit including: a composition in the form of gas, containing oxygen gas; and a composition containing a perfluorocarbon.
(14B) The kit for pre-use preparation according to (13B), for use in increasing the blood oxygen partial pressure of a subject.
(15B) The kit for pre-use preparation according to (13B) or (14B), for use in decreasing the blood carbon dioxide partial pressure of a subject.
(16B) An administration device for intrarectal administration, the administration device including the composition according to any one of (1B) to (4B) and (6B) to (12B).
(17B) An administration controller for oral administration, nasogastric administration, or intrarectal administration of a perfluorocarbon dissolving oxygen therein or oxygen, the administration controller including: a delivering unit configured to deliver the perfluorocarbon dissolving oxygen therein or oxygen to a tube; and a controlling unit configured to control the rate of delivery from the delivering unit on the basis of the blood oxygen saturation of a subject and the intraintestinal pressure of the rectum of the subject.
(18B) The administration controller according to (17B), further including a receiving unit configured to receive information on oxygen saturation from a blood oxygen monitor and intraintestinal pressure of the rectum.
(19B) The administration controller according to (17B) or (18B), including:
   a controlling unit configured to control the rate of delivery from the delivering unit on the basis of the blood oxygen saturation of the subject, wherein
   (A) the controlling unit refers to information on oxygen saturation received by a receiving unit, and if the oxygen saturation has reached lower than a predetermined value or a predetermined value or lower, the controlling unit sends a signal to increase the rate of delivery to the delivering unit; and/or
   (B) the controlling unit refers to information on oxygen saturation received by a receiving unit, and if the oxygen saturation has reached a predetermined value or higher or higher than a predetermined value, the controlling unit sends a signal to decrease the rate of delivery to the delivering unit.
(20B) The administration controller according to any one of (17B) to (19B), further including:
   a controlling unit configured to control the rate of delivery from the delivering unit on the basis of the intraintestinal pressure of the rectum of the subject, wherein
   (C) the controlling unit refers to information on intraintestinal pressure of the rectum received by a receiving unit, and if the intraintestinal pressure has reached lower than a predetermined value or a predetermined value or lower or if the intraintestinal pressure has increased during liquid delivery, the controlling unit stops sending a signal to increase the rate of delivery to the delivering unit, or sends a signal to decrease the rate of delivery to the delivering unit.
(21B) A method for administering oxygen to a subject, the method including:
   administering a pharmaceutical composition containing a perfluorocarbon dissolving oxygen therein to the subject through oral administration, nasogastric administration, or intrarectal administration.
(22B) The method according to (21B), including:
   administering a perfluorocarbon dissolving oxygen therein to the subject through oral administration, nasogastric administration, or intrarectal administration with dose control using an administration controller, wherein
   the administration controller is an administration controller for administering a perfluorocarbon dissolving oxygen therein or oxygen, and includes: a delivering unit configured to deliver the perfluorocarbon dissolving oxygen therein or oxygen to a tube; and a controlling unit configured to control the rate of delivery from the delivering unit on the basis of the blood oxygen saturation of the subject and/or the intraintestinal pressure of the rectum of the subject.
(23B) The method according to (22B) or (23B), wherein the rectum is a rectum.
(24B) The composition according to any one of (1B) to (4B) and (6B) to (12B), for administering a perfluorocarbon dissolving oxygen therein to the subject through oral administration, nasogastric administration, or intrarectal administration with dose control using an administration controller, wherein
   the administration controller is an administration controller for administering a perfluorocarbon dissolving oxygen therein or oxygen, and includes: a delivering unit configured to deliver the perfluorocarbon dissolving oxygen therein or oxygen to a tube; and a controlling unit configured to control the rate of delivery from the delivering unit on the basis of the blood oxygen saturation of the subject and/or the intraintestinal pressure of the rectum of the subject.
(25B) An administration controller for administering oxygen gas to a rectum, the administration controller including: a gas-delivering unit configured to deliver oxygen to a tube; and a controlling unit configured to control the rate of gas delivery from the gas-delivering unit on the basis of the blood oxygen saturation of a subject and/or the intraintestinal pressure of the rectum (preferably the rectum) of a subject.
(26B) The administration controller according to (25B), further including a receiving unit configured to receive information on oxygen saturation from a blood oxygen monitor and/or intraintestinal pressure of the rectum.
(27B) The administration controller according to (25B) or (26B), including:
   a controlling unit configured to control the rate of gas delivery from the gas-delivering unit on the basis of the blood oxygen saturation of the subject, wherein
   (A) the controlling unit refers to information on oxygen saturation received by a receiving unit, and if the oxygen saturation has reached lower than a predetermined value or a predetermined value or lower, the controlling unit sends a signal to increase the rate of gas delivery to the gas-delivering unit; and/or
   (B) the controlling unit refers to information on oxygen saturation received by a receiving unit, and if the oxygen saturation has reached a predetermined value or higher or higher than a predetermined value, the controlling unit sends a signal to decrease the rate of gas delivery to the gas-delivering unit.
(28B) The administration controller according to any one of (25B) to (27B) including:
   a controlling unit configured to control the rate of gas delivery from the gas-delivering unit on the basis of the intraintestinal pressure of the rectum of a subject, wherein
   (C) the controlling unit refers to information on intraintestinal pressure of the rectum received by a receiving unit, and if the intraintestinal pressure has reached lower than a predetermined value or a predetermined value or lower, the controlling unit stops sending a signal to increase the rate of gas delivery to the gas-delivering unit, or sends a signal to decrease the rate of gas delivery to the gas-delivering unit.
(29B) A pharmaceutical composition containing a perfluorocarbon dissolving oxygen therein, wherein the pharmaceutical composition is administered to a human through oral administration.
(30B) A pharmaceutical composition containing a perfluorocarbon dissolving oxygen therein, wherein the pharmaceutical composition is administered to a human through nasogastric administration.
(31B) A pharmaceutical composition containing a perfluorocarbon dissolving oxygen therein, wherein the pharmaceutical composition is administered to a human through intrarectal administration.
(32B) The pharmaceutical composition according to any one of (29B) to (31B), wherein the perfluorocarbon has an oxygen saturation of 50% or higher, preferably of 60% or higher, more preferably of 70% or higher, further preferably of 80% or higher, furthermore preferably of 90% or higher, especially preferably of 95% or higher.
(33B) The pharmaceutical composition according to any one of (29B) to (32B), wherein the administration is administration with an administration device.
(34B) The pharmaceutical composition according to any one of (29B) to (33B), wherein the perfluorocarbon is a perfluorocarbon maintained in the atmosphere.
(35B) The pharmaceutical composition according to any one of (29B) to (33B), wherein the perfluorocarbon has a dissolved oxygen content higher than that in the atmosphere; for example, the perfluorocarbon can be used after further dissolving oxygen therein.
(36B) The pharmaceutical composition according to any one of (29B) to (35B), wherein the human has hypoxemia.
(37B) The pharmaceutical composition according to any one of (29B) to (36B), wherein the human has a blood carbon dioxide partial pressure higher than 45 Torr.
(38B) The pharmaceutical composition according to any one of (29B) to (37B), wherein the human has respiratory failure.
(39B) The pharmaceutical composition according to (38B), wherein the human has pneumonia involving respiratory failure.
(40B) The pharmaceutical composition according to any one of (29B) to (38B), wherein the human has asthma.
(41B) The pharmaceutical composition according to any one of (29B) to (38B), wherein the human has chronic obstructive pulmonary disease (COPD).
(42B) The pharmaceutical composition according to any one of (29B) to (38B), wherein the human has any disease listed in Table 1.

### Brief Description of Drawings

[Figure 1A] Figure 1A shows an adult loach under anesthesia with isoflurane, and the overall image and cross-section of the intestine of the loach.
[Figure 1B] Figure 1B shows a mouse under anesthesia with isoflurane, and the overall image and cross-section of the intestine of the mouse.
[Figure 1C] Figure 1C shows hematoxylin staining of the normal posterior intestine of a loach and the intestines of mice after intestinal gas ventilation (IGV). It is demonstrated that the distance between the intestinal lumen and microvessels was shorter than that in the control group.
[Figure 1D] Figure 1D shows results of quantitative RT-PCR analysis of *Vegfa* and *Anxal* genes from abraded distal intestines of mice. Data are represented as multiples of a value for anterior intestines subjected to control treatment. Each value is average ± SE (n = 3). Differences were analyzed with ANOVA and Tukey post hoc test. *p < 0.05 is against control treatment.
[Figure 1E] Figure 1E shows results of immunochemical staining with Hypoxyprobe^{™} for mice with neither mucosal abrasion nor IGV. This staining demonstrated that Hypoxyprobe^{™}-positive cells were abundant in intestinal epithelial regions under highly hypoxic condition. In the intestine of mice placed under highly hypoxic condition with mucosal abrasion and intestinal gas ventilation (IGV), weakly Hypoxyprobe^{™}-positive cells were present in intestinal epithelial regions, indicating therapeutic effect and improvement for hypoxic regions. PI: posterior intestine.
[Figure 2] Figure 2 shows results of treatment of lethal hypoxemia by intestinal gas ventilation through systemic oxygenation. Panel A shows survival rates in a control group, an intestinal gas ventilation group, and an intestinal gas ventilation group (IGV) subjected to mucosal abrasion under 8% critical hypoxic condition. Panel B shows oxygen partial pressure in the inferior vena cava with intestinal gas ventilation. Panel C shows oxygen partial pressure in the left ventricle of the heart with intestinal ventilation.

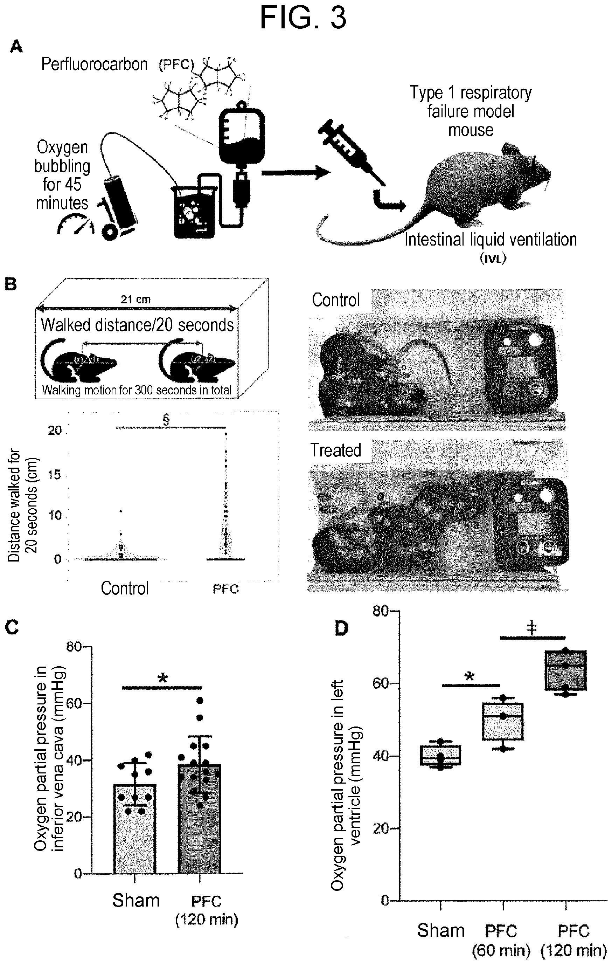

[Figure 3] Figure 3 shows results of treatment of lethal hypoxemia by intraintestinal administration of O₂-loaded PFC without mucosal abrasion. Panel A shows a schematic diagram of the experimental procedure. Panel B shows a schematic diagram of an experimental procedure of behavior tracking and photographing for mice. Walk distance was statistically longer in the treated group than in the control group (control group: 0.408 ± 1.02 cm, treated group: 3.34 ± 4.05 cm, P < 0.0001). Panel C shows improvement of oxygenation in the inferior vena cava by fluorocarbon therapy. Panel D shows improvement of oxygenation of the left ventricle of the heart under hypoxic condition by intestinal gas ventilation therapy (intestinal fluid ventilation therapy) using a perfluorocarbon.
[Figure 4A] Figure 4A shows results of treatment of hypoxemia under different intestinal ventilation protocols. Shown is venous oxygen partial pressure under conditions with or without intestinal gas ventilation and mucosal abrasion. Pressure values for intestinal gas ventilation involving moderate mechanical mucosal abrasion were significantly higher than those in the intestinal gas ventilation group without mucosal abrasion (PvO₂ 52.0 ± 7.88 mmHg and 38.4 ± 11.3 mmHg, p = 0.042).
[Figure 4B] Figure 4B shows distance between the intestinal lumen and the muscular mucosa.
[Figure 4C] Figure 4C shows quantitative RT-PCR analysis of *Spon1* and *Glud1* genes from distal intestines of mice subjected to mucosal abrasion. Data are represented as multiples of a value for anterior intestines subjected to control treatment. Each value is represented as average ± SE (n = 3). No statistical difference was found between the control group and any of the treated groups.
[Figure 4D] Figure 4D shows estimated scores with Hypoxyprobe^{™}.
[Figure 4E] Figure 4E shows temporal variation of the oxygen partial pressure of a perfluorocarbon (PFC) bubbled with pure oxygen. The average pressure of oxygen in the perfluorocarbon after 120 minutes was 438 ± 19.9 mmHg.
[Figure 5] Figure 5 shows a schematic diagram of a common enema including a fig-shaped container.
[Figure 6] Figure 6 shows a schematic diagram of a common enema including a bellows container.
[Figure 7] Figure 7 shows a schematic diagram of an administration device of the present invention configured to administer to a tube of intestinal placement type.
[Figure 8] Figure 8 shows a schematic diagram of an administration device of the present invention that is capable of simultaneously performing both liquid delivery and gas delivery.
[Figure 9] Figure 9 shows results of treatment of hypoxemia under an intestinal ventilation protocol. Figure 9 compares the effect of a research-grade PFC and that of a clinical-grade PFC.
[Figure 10] Figure 10 shows the influence of the presence or absence of clamping the inferior vena cava or the portal vein on oxygen supply to blood under an intestinal ventilation protocol.
[Figure 11] Figure 11 shows results of treatment of hypoxemia under an intestinal ventilation protocol with use of a gelled PFC.
[Figure 12A] Figure 12A shows the appearance and tissue images of lungs of an acute respiratory distress syndrome (ARDS) pig model.
[Figure 12B] Figure 12B shows results of treatment for an acute respiratory distress syndrome (ARDS) pig model under an intestinal ventilation protocol.
[Figure 13] Figure 13 shows results of treatment of hypoxemia under an intestinal ventilation protocol with use of perflubron (PFB) as a PFC.
[Figure 14A] Figure 14A shows that a mouse to which a bubbled PFC had been orally administered had the intestinal tract swollen by the PFD.
[Figure 14B] Figure 14B shows that it is possible to recover a PFC orally administered to a mouse from the intestinal tract of the mouse.
[Figure 14C] Figure 14C shows enhancement of oxygen partial pressure and decrease of carbon dioxide partial pressure in the left ventricular blood of mice to which a PFC dissolving oxygen therein was orally administered.

### Description of Embodiments

Herein, the "subject" is a mammal, in particular, a primate such as a human, a tetrapod such as a dog, a cat, a hamster, a guinea pig, a horse, a bovine, a sheep, a pig, a camel, and a goat, or a bird such as a chicken. In particular, the subject is a human.

Herein, "perfluorocarbon" is a molecule formed by substituting all the hydrogen atoms in a hydrocarbon with fluorine atoms. The perfluorocarbon can contain a linear alkyl, a branched alkyl, or a cycloalkyl. One or more carbon atoms in the perfluorocarbon may be substituted with atoms selected from the group consisting of O, N, and S. Perfluorocarbon (PFC) is known to have high dissolution capacity for oxygen. Perfluorocarbon can be liquid at normal temperature. Perfluorocarbons are capable of dissolving oxygen gas approximately 20 times or more than water does.

Herein, "oxygen" is O₂. Oxygen gas exhibits high solubility to liquid perfluorocarbon. In general, oxygen accounts for approximately 21% of the atmosphere.

Herein, "oxygen therapy" refers to treatment to supply oxygen to a subject for the purpose of ameliorating respiratory failure in the subject.

Herein, "respiratory failure" is defined as condition such that abnormal values of arterial blood gas are exhibited, which makes the living body incapable of performing normal functions. Among classes of respiratory failure, hypoxic respiratory failure (hypoxemia) refers to respiratory system functional disorder such that the arterial blood oxygen partial pressure (PaO₂) in inhaling room air is 60 mmHg (approximately 8,000 Pa) or lower, or the abnormal condition corresponding to the functional disorder. Respiratory failure is roughly classified into type I respiratory failure and type II respiratory failure. Type I respiratory failure is respiratory failure such that PaO₂ is 45 mmHg (approximately 6,000 Pa) or lower, and type II respiratory failure is respiratory failure such that PaO₂ is higher than 45 mmHg (approximately 6,000 Pa). Quasi-respiratory failure refers to condition such that PaO₂ is higher than 60 mmHg (approximately 8,000 Pa) and 70 mmHg (approximately 9,333 Pa) or lower. Here, 1 mmHg is equivalent to 1 Torr, or 101,325/760 Pa. Respiratory failure can be caused by, for example, pneumonia (e.g., viral pneumonia due to influenza virus, measles virus, coronavirus, or varicella virus, etc., and bacterial pneumonia due to *Haemophilus influenzae, Staphylococcus aureus,* or *Streptococcus pneumoniae,* etc., and atypical pneumonia due to a microorganism such as *Mycoplasma* spp. and *Chlamydia* spp.).

Herein, the term "gas" means a gaseous substance. Gas can take the form of gas or a form dissolved in liquid. However, the term gas herein means a gaseous substance in the form of gas, unless otherwise specified.

Herein, the term "oxygen-containing liquid" means liquid dissolving O₂ therein.

Herein, the term "intestinal tract" means the small intestine and large intestine. The large intestine includes the colon and the rectum. Herein, administration into a large intestine and intrarectal administration do not exclude administering from the outside of a body through a hole opened in the large intestine or rectum, but can be preferably administration via the anus. The administration into a large intestine or intrarectal administration can be preferably performed by using an administration device such as an enema device. The administration into a large intestine or intrarectal administration may be trans-artificial-anus administration (e.g., via a stoma).

Herein, oral administration is administration via the mouth. The oral administration can be performed through common oral administration, without limitation. Administration can also be performed through nasogastric administration, for example, with a device of nasal insertion type (e.g., a nasogastric tube). In the nasogastric administration, an object to be administered can be directly administered to the stomach through a nasogastric tube that has reached to the stomach.

The present invention provides, as a first aspect, a pharmaceutical composition for oral administration, nasogastric administration, trans-fistula gastric administration, or intraintestinal administration (in particular, a pharmaceutical composition for administration into a large intestine, more preferably a pharmaceutical composition for intrarectal administration), the pharmaceutical composition containing a perfluorocarbon dissolving oxygen therein. In this aspect, the pharmaceutical composition is in the form of liquid. Hereinafter, description of the pharmaceutical composition for intraintestinal administration is also applicable to pharmaceutical compositions for oral administration, nasogastric administration, and trans-fistula gastric administration.

Perfluorocarbon is liquid at normal temperature. Perfluorocarbon and other highly fluorinated liquids have high affinity with gasses, and, for example, are capable of dissolving oxygen approximately 20 times or more than water does. In addition, PFC liquids are inert, and have no or little toxicity (Riess, (1984) Artificial Organs, 8: 44-56). Mammals can respire with an oxygenated perfluorocarbon without long-term influence, and recover air breathing after that (Modell et al., (1970) Federation Proc., 29: 1731-1736; Modell et al., (1976) Chest, 69: 79-81). Here, the term oxygenated perfluorocarbon refers to a perfluorocarbon in which oxygen has been dissolved by oxygen bubbling for the liquid of the perfluorocarbon. The fluorocarbon molecule to be used in the present invention can have various structures including a linear or branched, or cyclic structure (Riess, (1984) Artificial Organs, 8: 44-56). Preferably, the fluorocarbon has approximately 2, 3, 4, or 5 to approximately 10, 12, or 14 carbon atoms. Many fluorocarbons are available as a fluorocarbon applicable in the present invention, and the perfluorocarbon may have a certain degree of unsaturation, may have a bromine or hydrogen atom, and may be an amine derivative; however, it is preferable that all the hydrogen atoms in such a fluorocarbon be substituted with fluorine atoms. Fluorocarbons include bis(F-alkyl)ethanes such as C₄F₉=CH₄CF₉ (occasionally expressed as "F-44E"), i-C₃F₉=CHC₆F₁₃ ("F-136E"), and C₆F₁₃CH=CHC₆CF₁₃ ("F-66E"); cyclic fluorocarbons such as C₁₀F₁₈ ("F-decalin", "perfluorodecalin" or "FDC"), F-adamantane ("FA"), F-methyladamantane ("FMA"), F-1,3-dimethyladamantane ("FDMA"), F-di- or F-trimethylbicyclo[3.3.1]nonane ("nonane"); perfluorinated amines such as F-tripropylamine ("FTPA") and F-tri-butylamine ("FTBA"), F-4-methyloctahydroquinolizine ("FMOQ"), F-n-methyldecahydroisoquinoline ("FMIQ"), F-n-methyldecahydroquinoline ("FHQ"), and F-n-cyclohexylprolidine ("FCHP"); and F-2-butyltetrahydrofuran ("FC-75" or "RM101), perfluorobutane, perfluoropropane, perfluoropentane, perfluorohexane, perfluoroheptane, and perfluorooctane. Moreover, linear and branched isomers of them are both contemplated. Other appropriate fluorocarbons selectable include brominated perfluorocarbons such as 1-bromo-heptadecafluorooctane (C₈F₁₇Br, "PFOB", or perflubron) and 1-bromopenta-decafluorohexane (C₆F₁₃Br, "PFHB"). Other brominated fluorocarbons are disclosed in U.S. Patent No. 3,975,512 by Long. Fluorocarbons having a non-fluorine substituent such as perfluorooctyl chloride and hydrogenated perfluorooctyl, and fluorocarbons having a different number of carbon atoms, such as 6 to 12 carbon atoms, are also contemplated. Other fluorocarbons contemplated according to the present invention include perfluoroalkylated ethers and polyethers such as (CF₃)₂CFO(CF₂CF₂)₂OCF(CF₃)₂, (CF₃)₂CFO(CF₂CF₂)₃OCF(CF₃), (CF₃)CFO(CF₂CF₂)F, (CF₃)₂CFO(CF₂CF₂)₂F, and (C₃F₁₃)₂O. Furthermore, fluorocarbon-hydrocarbon compounds such as compounds represented by the general formula CₙF₂ₙ₊₁C_{n'}F_{2n'+1,} CₙF₂ₙ₊₁₀C_{n'}F_{2n'+1,} or CₙF₂ₙ₊₁CF=CHC_{n'}F_{2n'+1}, wherein n and n' are the same or different, and are each about 1 to about 10 (provided that the compounds are liquid at room temperature) are included. Such compounds include C_{B}F₁₇C₂H₅ and C₆F₁₃CH=CHC₆H₁₃.

In a certain preferred embodiment of the present invention, the perfluorocarbon (PFC) is, for example, one or more selected from the group consisting of perfluorooctane, perfluorobutylperfluorotetrahydrofuran, perfluoro-1-isopropoxyhexane, perfluoro-1,4-diisopropoxybutane, and octadecafluorodecahydronaphthalene. In another or additional embodiment, the PFC in the composition is selected from perfluorodecalin (PFD; C₁₀F₁₈), perflubron (PFB; C₈BrF₁₇), perfluoro-1,3-dimethylcyclohexane, FC-75, perfluorooctane, and perfluoro-octyl bromide. In some embodiments, the PFC is or contains a PFC having a cycloalkyl group such as perfluorodecalin, perfluoro-1,3-dimethylcyclohexane, and FC-75.

According to the present invention, the pharmaceutical composition for intraintestinal administration contains an effective amount of a perfluorocarbon dissolving oxygen therein. Here, an effective amount is such an amount that once being administered through intraintestinal administration (preferably administration into a large intestine, more preferably intrarectal administration), the perfluorocarbon sends oxygen from the mucosa of an intact intestinal tract into blood to increase the oxygen partial pressure of the blood (e.g., in the artery, in the vein, in the pulmonary artery, in the pulmonary vein, or in the left ventricle or in the right ventricle).

According to the present invention, the perfluorocarbon dissolving oxygen therein has an oxygen partial pressure higher than blood oxygen partial pressure. In an adult human, the oxygen saturation is about 98% at an oxygen partial pressure of 100 mmHg, about 95% at 80 mmHg, and about 90% at 60 mmHg. Accordingly, it is preferable for the perfluorocarbon dissolving oxygen therein to have, for example, an oxygen partial pressure of 100 mmHg or higher, 150 mmHg or higher, 200 mmHg or higher, or 250 mmHg or higher. By virtue of this, use of the perfluorocarbon is expected to lead to successful achievement of intestinal gas ventilation. In a certain preferred embodiment, oxygen can be artificially dissolved in the perfluorocarbon. For example, dissolution of oxygen in the perfluorocarbon can be performed even by bubbling a solution of the perfluorocarbon with oxygen gas. For example, bubbling with oxygen gas can be performed until the oxygen concentration of the perfluorocarbon saturates, or reaches 50% or higher, 60% or higher, 70% or higher, 80% or higher, or 90% or higher of the saturated oxygen concentration. Bubbling may be performed by using an apparatus configured to bubble a solution of the perfluorocarbon with oxygen gas. The saturated concentration is that at room temperature under the atmospheric pressure. For example, according to the present invention, a composition (pharmaceutical composition) in the form of gas, containing oxygen gas can be dissolved in a perfluorocarbon before administration and then administered into the intestine of a subject. According to the present invention, for another example, the pharmaceutical composition containing a perfluorocarbon can have oxygen dissolved therein, or preferably be allowed to dissolve oxygen therein before administration and then administered into the intestine (preferably into the large intestine, in particular, into the rectum) of a subject. Alternatively, the pharmaceutical composition containing a perfluorocarbon dissolving an effective amount of oxygen therein can be provided and administered into the intestine of a subject.

According to the present invention, for example, the pharmaceutical composition containing a perfluorocarbon can have oxygen dissolved therein, or preferably be allowed to dissolve oxygen therein before administration and then administered to a subject through oral administration. Alternatively, the pharmaceutical composition containing a perfluorocarbon dissolving an effective amount of oxygen therein can be provided and administered to a subject through oral administration.

According to the present invention, for example, the pharmaceutical composition containing a perfluorocarbon can have oxygen dissolved therein, or preferably be allowed to dissolve oxygen therein before administration and then administered to a subject through nasogastric administration. Alternatively, the pharmaceutical composition containing a perfluorocarbon dissolving an effective amount of oxygen therein can be provided and administered to a subject through nasogastric administration. The nasogastric administration can be administration into the stomach.

In a certain preferred embodiment, the perfluorocarbon has an oxygen partial pressure of 250 mmHg or higher in the atmosphere (oxygen concentration: approximately 210), and thus a perfluorocarbon maintained in the atmosphere (or allowed to have oxygen dissolved therein in the atmosphere) is applicable in the present invention.

Once the pharmaceutical composition of the present invention for intraintestinal administration (preferably the pharmaceutical composition for administration into a large intestine, more preferably the pharmaceutical composition for intrarectal administration) is administered into the intestinal tract (preferably into the large intestine) of a subject, the perfluorocarbon causes migration of dissolved oxygen into blood through the permeation of oxygen dissolved in the perfluorocarbon into the mucosa of the intestinal tract. This improves the blood oxygen partial pressure of a subject. Accordingly, the pharmaceutical composition of the present invention for intraintestinal administration (preferably the pharmaceutical composition for administration into a large intestine, more preferably the pharmaceutical composition for intrarectal administration) can be used for treating hypoxemia. The subject for administration of the pharmaceutical composition can be a patient having an arterial blood oxygen saturation of 90 or lower, 85 or lower, 80 or lower, 75 or lower, 70 or lower, 65 or lower, or 60 or lower. Once the pharmaceutical composition of the present invention for intraintestinal administration (preferably the pharmaceutical composition for administration into a large intestine, more preferably the pharmaceutical composition for intrarectal administration) is administered into the intestinal tract (preferably into the large intestine) of a subject, gas (in particular, carbon dioxide) dissolved in the blood is adsorbed on the perfluorocarbon to decrease the blood carbon dioxide concentration. Accordingly, the pharmaceutical composition of the present invention for intraintestinal administration (preferably the pharmaceutical composition for administration into a large intestine, more preferably the pharmaceutical composition for intrarectal administration) can be used for decreasing blood gas concentration (in particular, blood carbon dioxide concentration). The subject for the pharmaceutical composition can be, for example, a patient having an arterial blood carbon dioxide partial pressure (PaCO₂) of >45 mmHg. Diseases involving pathological condition with airway obstruction such as asthma and chronic obstructive pulmonary disease (COPD) can cause increase in blood carbon dioxide partial pressure because of failure in sufficient excretion of carbon dioxide. Accordingly, the pharmaceutical composition of the present invention can be used for treating such a disease (or a condition) involving increase in blood carbon dioxide partial pressure (e.g., higher than 45 mmHg). In a certain embodiment, the pharmaceutical composition of the present invention for intraintestinal administration can be administered through single administration, multiple administration, or continuous administration. The continuous administration may be performed by administering a fresh PFC while the PFC previously administered is recovered. In using for decreasing carbon dioxide partial pressure, the PFC does not need to be subjected to additional oxygenation in advance.

Hypoxemia is induced in subjects having respiratory failure. Accordingly, the pharmaceutical composition of the present invention for intraintestinal administration (preferably the pharmaceutical composition for administration into a large intestine, more preferably the pharmaceutical composition for intrarectal administration) can be used for treating respiratory failure in a subject. In addition, the pharmaceutical composition of the present invention for intraintestinal administration (preferably the pharmaceutical composition for administration into a large intestine, more preferably the pharmaceutical composition for intrarectal administration) can be used for treating hypoxemia in a subject having respiratory failure. Thus, the pharmaceutical composition of the present invention for intraintestinal administration (preferably the pharmaceutical composition for administration into a large intestine, more preferably the pharmaceutical composition for intrarectal administration) can be for use in supplying oxygen to the blood of a subject.

The pharmaceutical composition of the first aspect does not need mucosal abrasion for the intestinal tract of a subject for administration.

The pharmaceutical composition of the present invention for intraintestinal administration is a liquid formulation. The pharmaceutical composition of the present invention for intraintestinal administration may further contain a pharmaceutically acceptable diluent.

In a certain embodiment, the pharmaceutical composition containing a perfluorocarbon dissolving oxygen therein can be administered to a human through oral administration. In a certain embodiment, the pharmaceutical composition containing a perfluorocarbon dissolving oxygen therein can be administered to the stomach of a human through nasogastric administration. In a certain embodiment, the pharmaceutical composition containing a perfluorocarbon dissolving oxygen therein can be administered to a human through intrarectal administration. In a certain preferred embodiment, the administration can increase the blood oxygen partial pressure of a human and/or decrease the blood carbon dioxide partial pressure.

In a certain embodiment, the pharmaceutical composition containing a perfluorocarbon dissolving oxygen therein can be a perfluorocarbon maintained in the atmosphere, and can be administered to a human through oral administration. In a certain embodiment, the pharmaceutical composition containing a perfluorocarbon dissolving oxygen therein can be a perfluorocarbon maintained in the atmosphere, and can be administered to the stomach of a human through nasogastric administration. In a certain embodiment, the pharmaceutical composition containing a perfluorocarbon dissolving oxygen therein can be a perfluorocarbon maintained in the atmosphere, and the pharmaceutical composition containing a perfluorocarbon dissolving oxygen therein can be administered to a human through intrarectal administration. In a certain preferred embodiment, the administration can increase the blood oxygen partial pressure of a human and/or decrease the blood carbon dioxide partial pressure.

In a certain embodiment, the pharmaceutical composition containing a perfluorocarbon dissolving oxygen therein has been artificially oxygenated. In a preferred embodiment, the artificial oxygenation can be performed by oxygen bubbling for a liquid containing the perfluorocarbon. In a certain embodiment, such a pharmaceutical composition can be administered to a human through oral administration. In a certain embodiment, such a pharmaceutical composition can be administered to the stomach of a human through nasogastric administration. In a certain embodiment, such a pharmaceutical composition can be administered to a human through intrarectal administration. In a certain preferred embodiment, the administration can increase the blood oxygen partial pressure of a human and/or decrease the blood carbon dioxide partial pressure.

In a certain preferred embodiment, the perfluorocarbon can be perfluorodecalin. In a certain preferred embodiment, the perfluorocarbon can be perflubron.

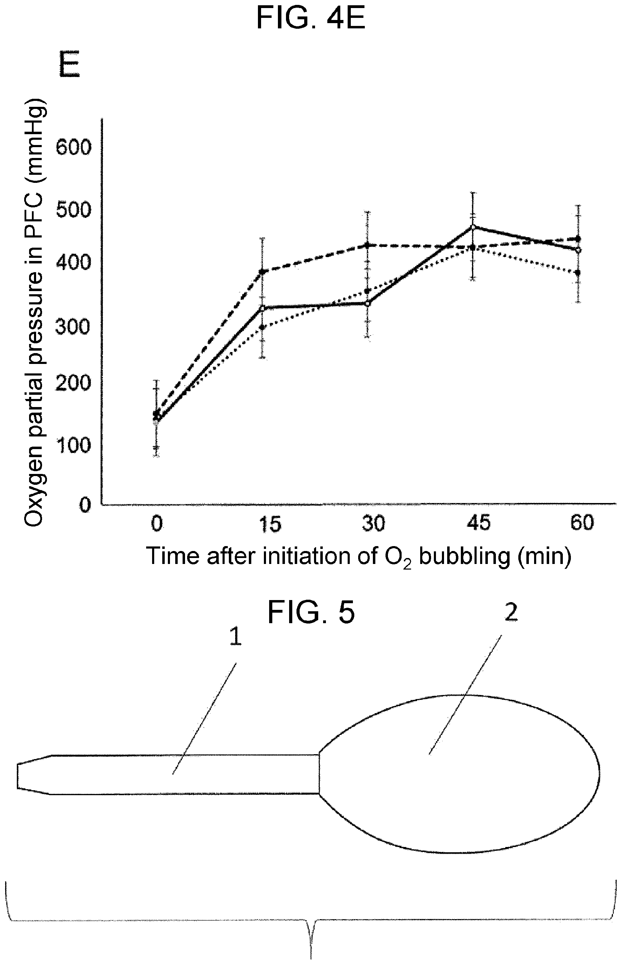

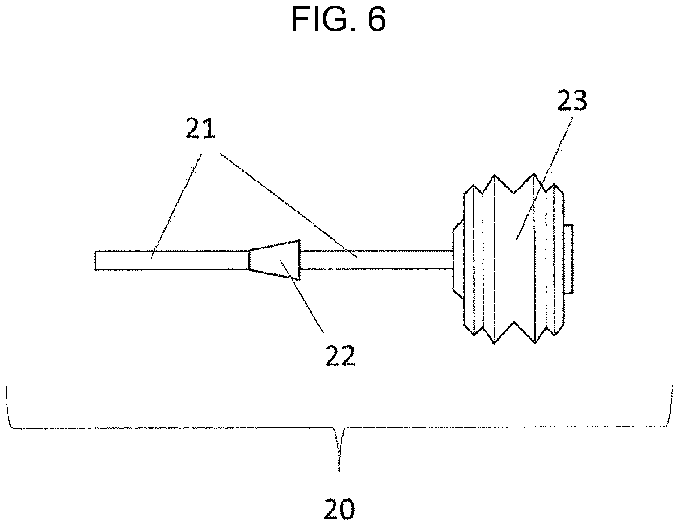

The pharmaceutical composition of the present invention for intraintestinal administration may be installed in an administration device for intestinal administration (preferably an administration device for administration into a large intestine, more preferably an administration device for intrarectal administration). The administration device for intestinal administration (preferably an administration device for administration into a large intestine, more preferably an administration device for intrarectal administration) can be an enema having a fig-shaped container (e.g., see Figure 5) or an enema having a bellows container (e.g., see Figure 6).

As illustrated in Figure 5, the enema 10 having a fig-shaped container has a nozzle 1 and a fig-shaped container 2. The pharmaceutical composition of the present invention for intraintestinal administration (preferably the pharmaceutical composition for administration into a large intestine, more preferably the pharmaceutical composition for intrarectal administration) is stored within the fig-shaped container 2. The enema 10 having a fig-shaped container may further include a check valve in the nozzle 1. In using the enema 10 having a fig-shaped container, a user can push out a content in the fig-shaped container 2 into the intestinal tract by pinching the fig-shaped container 2, which has elasticity.

As illustrated in Figure 6, the enema 20 having a bellows container has a nozzle 21 and a bellows container 23, and preferably includes a check valve 22. The pharmaceutical composition of the present invention for intraintestinal administration (preferably the pharmaceutical composition for administration into a large intestine, more preferably the pharmaceutical composition for intrarectal administration) is stored within the bellows container 23. In using the enema 20 having a bellows container, a user can push out a content in the bellows container 23 into the intestinal tract (preferably into the large intestine, more preferably into the rectum) by pinching the bellows container 23, which has elasticity.

Thus, according to the present invention, the pharmaceutical composition of the present invention can be formulated as an administration device for intraintestinal administration (preferably an administration device for administration into a large intestine, more preferably an administration device for intrarectal administration). The present invention provides an administration device for intestinal administration (preferably an administration device for administration into a large intestine, more preferably an administration device for intrarectal administration), the administration device including the pharmaceutical composition of the present invention.

The pharmaceutical composition of the present invention can be administered to a subject by using a device for administration with a tube indwelling in the intestinal tract (e.g., in the large intestine, preferably in the rectum) such as an elemental diet tube (ED tube), an device for administration including a tube of nasal insertion type, such as a nasal-gastric tube and an ileus tube, and a device for administration including a tube of transanal insertion type such as an ileus tube). Alternatively, the pharmaceutical composition of the present invention may be directly administered to the stomach via a gastric fistula. Administration via a gastric fistula (trans-fistula gastric administration) can be performed by using a gastrostomy tube or the like. The administration into a large intestine or intrarectal administration may be trans-artificial-anus administration (e.g., via a stoma).

The present invention provides, as a second aspect, a pharmaceutical composition in the form of gas, containing oxygen gas, for intestinal administration (preferably for administration into a large intestine, more preferably for intrarectal administration). The pharmaceutical composition of the present invention in the form of gas for intestinal administration (preferably for administration into a large intestine, more preferably for intrarectal administration) does not contain a perfluorocarbon. The pharmaceutical composition of the present invention in the form of gas for intestinal administration (preferably for administration into a large intestine, more preferably for intrarectal administration) contains a sufficient concentration and amount of oxygen for improving the blood oxygen partial pressure of a subject once being administered to the subject through intraintestinal administration. The pharmaceutical composition in the form of gas has a gas composition suitable for pharmaceutical applications of the present invention.

The pharmaceutical composition of the present invention according to the second aspect can be preferably administered to a subject having an intestinal tract (preferably a large intestine, in particular, a distal intestine, or a rectum) subjected to mucosal removal. Here, the meaning of mucosal removal is that at least part (i.e., the entire or part of an area to be in contact with the pharmaceutical composition) of the intestinal tract has been subjected to mucosal removal or abrasion. By virtue of this, the blood oxygen partial pressure of a subject can be enhanced by administering oxygen in the form of gas from the intestinal tract, without use of a perfluorocarbon.

Physicians can appropriately apply mucosal removal with considering the age, body weight, body height, sex, etc., of a subject.

The pharmaceutical composition of the present invention according to the second aspect can be preferably administered to a subject having the intestinal tract (preferably the large intestine, more preferably the rectum) coated with a PFC. Here, the meaning of having the intestinal tract (preferably the large intestine) coated with a PFC is that at least part (i.e., the entire or part of an area to be in contact with the pharmaceutical composition) of the intestinal tract (preferably the large intestine, more preferably the rectum) is coated with a PFC. Through sending oxygen into the intestinal tract (preferably the large intestine, more preferably the rectum) with the surface thereof coated with a PFC, oxygen dissolves in the PFC. The resulting action exerted, which is similar to that of the pharmaceutical composition of the present invention according to the first aspect, is expected to provide an effect to enhance the oxygen saturation of a subject. In the present aspect, a composition containing a perfluorocarbon for administration into a large intestine can be preferably used, and the intestinal tract of a subject can be coated with a perfluorocarbon.

The pharmaceutical composition of the present invention according to the second aspect can be used for treating hypoxemia. The pharmaceutical composition of the present invention according to the second aspect can be used for treating hypoxic ischemia.

Hypoxemia is induced in subjects having respiratory failure. Accordingly, the pharmaceutical composition of the present invention according to the second aspect can be used for treating respiratory failure in a subject. The pharmaceutical composition of the present invention according to the second aspect can be used for treating hypoxemia in a subject having respiratory failure. Thus, the pharmaceutical composition of the present invention according to the second aspect can be for use in supplying oxygen to the blood of a subject.

The pharmaceutical composition of the present invention according to the second aspect may contain other gas such as nitrogen in addition to oxygen, unless the gas harms.

The pharmaceutical composition of the present invention according to the second aspect can be administered to a subject by using a device for administration with a tube indwelling in the intestinal tract (preferably in the large intestine, more preferably in the rectum) such as an elemental diet tube (ED tube), a device for administration including a tube of nasal insertion type, a nasal-gastric tube and an ileus tube, and a device for administration including a tube of transanal insertion type such as an ileus tube. Alternatively, the pharmaceutical composition of the present invention may be directly administered to the stomach via a gastric fistula. Administration via a gastric fistula (trans-fistula gastric administration) can be performed by using a gastrostomy tube or the like. The administration into a large intestine or intrarectal administration may be trans-artificial-anus administration (e.g., via a stoma).

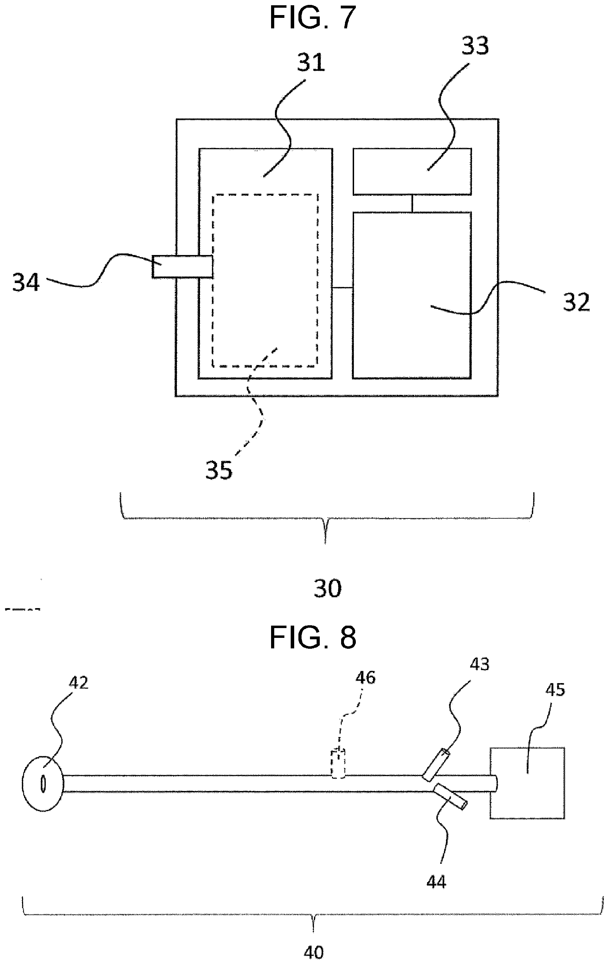

To administer the pharmaceutical composition of the present invention according to the first aspect and/or second aspect, an administration controller 30 having a tube indwelling in the intestinal tract can be used. As illustrated in Figure 7, the administration controller 30 can include: a delivering unit 31 configured to deliver the pharmaceutical composition of the present invention to a tube (toward the delivering unit 31); and a controlling unit 32 configured to control the rate of delivery from the delivering unit 31 on the basis of the blood oxygen saturation of a patient. Various methods for blood oxygen saturation (e.g., a measurement device to measure oxygen saturation in arterial blood from absorption wavelengths in light transmitted through a fingertip irradiated with red light and infrared light can be used) are known to those skilled in the art. Thus, the present invention provides an administration controller for the pharmaceutical composition of the present invention (hereinafter, referred to as "the administration controller of the present invention"), the administration controller including: a delivering unit 31 configured to deliver the pharmaceutical composition of the present invention to a tube; and a controlling unit 32 configured to control the rate of delivery from the delivering unit 31 on the basis of the blood oxygen saturation of a patient.

The administration controller of the present invention further includes a receiving unit 33 configured to receive information on oxygen saturation from a blood oxygen monitor. The controlling unit 32 can refer to information on oxygen saturation received by the receiving unit 33, and if the oxygen saturation has reached lower than a predetermined value or a predetermined value or lower, the controlling unit 32 can send a signal to increase the rate of delivery to the delivering unit 31. The controlling unit 32 can refer to information on oxygen saturation received by the receiving unit 33, and if the oxygen saturation has reached a predetermined value or higher or higher than a predetermined value, the controlling unit 32 can send a signal to decrease the rate of delivery to the delivering unit 31. In the administration controller of the present invention, the receiving unit 33 may further receive information on intraintestinal pressure (in particular, intraintestinal pressure in the large intestine) (here, the case that a receiving unit for information on oxygen saturation and a receiving unit for information on intraintestinal pressure are separately provided is not excluded). The controlling unit 32 can refer to information on intraintestinal pressure received by the receiving unit 33, and if the intraintestinal pressure has reached higher than a preset value or if the intraintestinal pressure begins to increase during liquid delivery, the controlling unit 32 can stop sending a signal to increase the delivery rate to the delivering unit, or send a signal to decrease the delivery rate to the delivering unit 31. If the intraintestinal pressure has reached lower than a preset value, the controlling unit 32 can send a signal to increase the delivery rate to the delivering unit 31. Alternatively, the delivering unit 31 can be configured to raise the delivery rate after receiving a signal to increase the delivery rate. Here, considering the oxygen saturation and/or intraintestinal pressure of a subject, the controlling unit 32 can decide whether to send a signal to increase the delivery rate or send a signal to decrease the delivery rate to the delivering unit 31. In addition, the delivering unit 31 can be configured to decrease the delivery rate after receiving a signal to decrease the delivery rate. The receiving unit 33 may be physically connected to a blood oxygen monitor via a cable or the like and receive signals through the cable or the like, or receive signals from an oxygen monitor via radio signals. The pharmaceutical composition of the present invention is passed from the delivering unit 31 through a delivery port 34, and ejected from the administration controller. The pharmaceutical composition ejected can be administered into the intestine via an administration device including a tube or an administration device connectable to the delivery port. Typically, such an administration device is configured separately from the administration controller, and can be connected to the administration controller in use. Thus, the administration controller 30 of the present invention can be operated to keep the blood oxygen saturation of a subject at a specific level or higher and/or to keep the intraintestinal pressure of a subject at a specific value or lower. Physicians can appropriately determine proper blood oxygen saturation and intraintestinal pressure, and when the intraintestinal pressure has reached a specific level or higher or the intraintestinal pressure has increased (or begins to increase), it is not preferable in normal cases to increase the intraintestinal administration rate of the drug even if the oxygen saturation is a preset value or lower. The present invention also provides a method for operating the administration controller of the present invention as described above. The administration device may include a sensor to measure intraintestinal pressure in the large intestine, and measurements acquired by the sensor can be sent to the receiving unit 33 of the administration controller. In a certain preferred embodiment, the intestinal tract is the large intestine, and more preferably the rectum.

The predetermined value on oxygen saturation can be, for example, a value of 50% or higher, 60% or higher, 70% or higher, 80% or higher, or 90% or higher, and can be, for example a value of 91% or higher, 92% or higher, 93% or higher, 94% or higher, 95% or higher, or 96% or higher.

The administration controller 30 of the present invention may have a storing unit 35 capable of storing the pharmaceutical composition of the present invention. The liquid-delivering unit 31 is capable of ejecting the drug stored in the storing unit 35 from the administration device via the liquid delivery port (or gas delivery port) 34. For example, a device for administration with a tube indwelling in the intestinal tract (preferably in the large intestine, more preferably in the rectum) can be connected to the liquid delivery port (or gas delivery port) 34 for use. Although the storing unit 35 is present in the administration device in the illustration, the storing unit 35 does not necessarily need to be included in the device, and is only required to be connected to the device. The administration device 30 does not need to include the storing unit 35; for example, a container (e.g., storage container) cartridge containing the pharmaceutical composition of the present invention may be connected to the administration device for use as the storage unit 35. In this case, once the drug in the container (e.g., storage container) cartridge containing the pharmaceutical composition of the present invention has been consumed, the container cartridge is to be replaced with a new container cartridge containing the pharmaceutical composition. Alternatively, the drug may be prepared before use by mixing oxygen gas and a perfluorocarbon and fed to the storing unit 35.

One or both of the pharmaceutical composition of the present invention according to the first aspect and the pharmaceutical composition of the present invention according to the second aspect can be administered to a subject. In this case, the pharmaceutical composition of the present invention according to the first aspect and the pharmaceutical composition of the present invention according to the second aspect may be simultaneously administered, or sequentially administered. In the case of sequential administration, administration of the pharmaceutical composition of the present invention according to the first aspect may be followed by administration of the pharmaceutical composition of the present invention according to the second aspect, and administration of the pharmaceutical composition of the present invention according to the second aspect may be followed by administration of the pharmaceutical composition of the present invention according to the first aspect. Alternatively, an administration cycle including administration of the pharmaceutical composition of the present invention according to the first aspect and the pharmaceutical composition of the present invention according to the second aspect may be carried out. In the case that administration of the pharmaceutical composition of the present invention according to the first aspect is followed by administration of the pharmaceutical composition of the present invention according to the second aspect, the effect of the pharmaceutical composition of the present invention according to the second aspect can be enhanced by administering the pharmaceutical composition of the present invention according to the second aspect through intraintestinal administration (preferably administration into a large intestine, more preferably intrarectal administration) while the PFC in the pharmaceutical composition of the present invention according to the first aspect is coating the inner wall of the intestinal tract (preferably the inner wall of the large intestine, more preferably the inner wall of the rectum).

If the pharmaceutical composition of the present invention according to the first aspect and the pharmaceutical composition of the present invention according to the second aspect are both administered with a device for administration via a tube indwelling in the intestinal tract, an administration device 40 can be used, as illustrated in Figure 8, the administration device 40 including: a tube 41; a balloon 42 configured to fix the tube in the rectum; a liquid administration port 43; a gas administration port 44; and a collection container 45 configured to collect excretions and so on in the rectum. The administration device 40 may have a pressure adjustment port 46 configured to adjust the pressure in the intestine. The pressure adjustment port 46 is capable of properly controlling the pressure in the intestine with a valve. For such an administration device, for example, a device that allows simultaneous administration of liquid and gas such as a Flexi-Seal can be used. The balloon has a small volume in insertion, and can be swollen with air or the like after the tube 41 has been inserted.

Use of the administration device 40 allows the pharmaceutical composition of the present invention according to the first aspect and the pharmaceutical composition of the present invention according to the second aspect to be simultaneously or sequentially administered to a subject.

Those skilled in the art could appropriately adjust the dose and time of administration of the pharmaceutical composition of the present invention on the basis of the blood oxygen partial pressure and oxygen saturation of a patient. In addition, those skilled in the art would be capable of using an additional ventilator and ECMO, etc., for treatment of respiratory failure in combination.

The present invention provides, as a further aspect,
a method for administering oxygen to a subject, the method including:
administering a pharmaceutical composition for intraintestinal administration containing a perfluorocarbon dissolving oxygen therein (e.g., the pharmaceutical composition of the first aspect) to the subject.

In a preferred embodiment, oxygen is administered into the intestinal tract, in particular, the large intestine or rectum of the subject.

The present invention provides, as a further aspect,
a method for treating hypoxemia in a subject in need thereof, the method including:
administering a pharmaceutical composition for intraintestinal administration containing a perfluorocarbon dissolving oxygen therein (e.g., the pharmaceutical composition of the first aspect) to the subject.

In a preferred embodiment, oxygen is administered into the intestinal tract, in particular, the large intestine or rectum of the subject.

In this aspect, the subject can have a disease, for example, listed in Table 1 in the following.

### [Table 1]

**Table 1: Examples of disease in subject as possible target of oxygen administration**

| **Disease category** | **Target diseases** |
|---|---|
| **Medical diseases** | Community-acquired pneumonia, aspiration pneumonia, nosocomial pneumonia, COVID-19 pneumonia (in particular, the number of patients with severe pneumonia or acute respiratory distress syndrome causative of acute respiratory failure as target disease: 30,000 per year) |
| **Diseases requiring emergency and anesthesia/critical care medicine** | Pathological condition possibly causative of difficult airway |
| | - infections (infectious pharyngeal/cranial inflammation, pneumonia, etc.) |
| | - tumors (laryngeal tumor, mediastinal tumor) |
| | - traumas (foreign body, spinal cord injury, basal skull fracture, etc.) |
| | - abnormalities of joints in head and neck (rheumatoid arthritis, etc.) |
| | - others (obesity, hypothyroidism, etc.) |
| **Indications for lung transplantation** | - idiopathic pulmonary arterial hypertension (the number of patients in 2014: 2299) |
| | - idiopathic pulmonary fibrosis (the number of patients: approximately 15000) |
| | - chronic obstructive pulmonary disease (261000 patients in 2014) |
| | - bronchiectasis (approximately 25000 patients), etc. |

The present invention provides, as a further aspect,
a method for treating respiratory failure in a subject in need thereof, the method including:
administering a pharmaceutical composition for intraintestinal administration containing a perfluorocarbon dissolving oxygen therein (e.g., the pharmaceutical composition of the first aspect) to the subject.

In a preferred embodiment, oxygen is administered into the intestinal tract, in particular, the large intestine or rectum of the subject.

In a certain embodiment of the present invention, the respiratory failure can be type I respiratory failure. In a certain embodiment, the respiratory failure can be pneumonia. In a certain embodiment, the pneumonia can be, for example, pneumonia due to an infection, and can be pneumonia due to a coronavirus infection (e.g., a viral infection caused by SARS-CoV-2).

The present invention provides, as a further aspect,
a method for decreasing the blood carbon dioxide partial pressure of a subject, the method including:
administering a perfluorocarbon or a perfluorocarbon dissolving oxygen therein (e.g., the pharmaceutical composition of the first aspect) to the intestinal tract of the subject with the mucosa of the intestinal tract removed.

In a preferred embodiment, the intestinal tract can be the large intestine or the rectum.

The present invention provides, as a further aspect,
a method for administering oxygen to a subject, the method including:
administering oxygen gas (e.g., the pharmaceutical composition of the second aspect) to the intestinal tract of the subject with the mucosa of the intestinal tract removed.

In a preferred embodiment, oxygen is administered to the large intestine or rectum of the subject.

The present invention provides, as a further aspect,
a method for treating hypoxemia in a subject in need thereof, the method including:
administering oxygen gas (e.g., the pharmaceutical composition of the second aspect) to the intestinal tract of the subject with the mucosa of the intestinal tract removed.

In a preferred embodiment, the intestinal tract can be the large intestine or the rectum.

The present invention provides, as a further aspect,
a method for treating respiratory failure in a subject in need thereof, the method including:
administering oxygen gas (e.g., the pharmaceutical composition of the second aspect) to the intestinal tract of the subject with the mucosa of the intestinal tract removed.

In a preferred embodiment, the intestinal tract can be the large intestine or the rectum.

In a certain embodiment of the present invention, the respiratory failure can be type 1 respiratory failure. In a certain embodiment of the present invention, the method of the present invention including administrating oxygen gas (e.g., the pharmaceutical composition of the second aspect) may further include removing the mucosa of the intestinal tract of the subject. In a preferred embodiment, the intestinal tract can be the large intestine or the rectum.

The present invention provides, as a further aspect,
a method for decreasing the blood carbon dioxide partial pressure of a subject, the method including:
administering oxygen gas (e.g., the pharmaceutical composition of the second aspect) to the intestinal tract of the subject with the mucosa of the intestinal tract coated with a perfluorocarbon.

In a preferred embodiment, the intestinal tract can be the large intestine or the rectum.

The present invention provides, as a further aspect,
a method for administering oxygen to a subject, the method including:
administering oxygen gas (e.g., the pharmaceutical composition of the second aspect) to the intestinal tract of the subject with the mucosa of the intestinal tract coated with a perfluorocarbon.

In a preferred embodiment, oxygen is administered to the large intestine or rectum of the subject.

The present invention provides, as a further aspect,
a method for treating hypoxemia in a subject in need thereof, the method including:
administering oxygen gas (e.g., the pharmaceutical composition of the second aspect) to the intestinal tract of the subject with the mucosa of the intestinal tract coated with a perfluorocarbon.

In a preferred embodiment, the intestinal tract can be the large intestine or the rectum.

The present invention provides, as a further aspect,
a method for treating respiratory failure in a subject in need thereof, the method including:
administering oxygen gas (e.g., the pharmaceutical composition of the second aspect) to the intestinal tract of the subject with the mucosa of the intestinal tract coated with a perfluorocarbon.

In a preferred embodiment, the intestinal tract can be the large intestine or the rectum.

In a certain embodiment of the present invention, the respiratory failure can be type 1 respiratory failure. In a certain embodiment of the present invention, the method of the present invention including administering oxygen gas (e.g., the pharmaceutical composition of the second aspect) may further include coating the mucosa of the intestinal tract of the subject with a perfluorocarbon. In a preferred embodiment, the intestinal tract can be the large intestine or the rectum.

The present invention provides, as a further aspect,
a method for decreasing the blood carbon dioxide partial pressure of a subject, the method including:
administering oxygen gas (e.g., the pharmaceutical composition of the second aspect) to the intestinal tract of the subject with the mucosa of the intestinal tract coated with a perfluorocarbon.

In a preferred embodiment, the intestinal tract can be the large intestine or the rectum.

In the above descriptions, coating of the mucosa of the intestinal tract can be entire or partial coating. The intestinal tract can be, in particular, the rectum, and coating can be performed for the entire or part of the mucosa of the rectum.

The present invention can provide, as a further aspect, a composition, containing oxygen gas, for use in any of the above methods.

The present invention can provide, as a further aspect, a composition containing a perfluorocarbon for use in any of the above methods.

The present invention can provide, as a further aspect, a composition containing a perfluorocarbon dissolving oxygen gas therein for use in any of the above methods.

The present invention provides, as a further aspect, use of a perfluorocarbon dissolving oxygen therein in manufacture of a pharmaceutical composition for intraintestinal administration (preferably for administration into a large intestine, more preferably for intrarectal administration). The pharmaceutical composition of the present invention for intraintestinal administration (preferably for administration into a large intestine, more preferably for intrarectal administration) can be used for treating hypoxemia. The pharmaceutical composition of the present invention for intraintestinal administration (preferably for administration into a large intestine, more preferably for intrarectal administration) can be used for treating respiratory failure in a subject. The pharmaceutical composition of the present invention for intraintestinal administration (preferably for administration into a large intestine, more preferably for intrarectal administration) can be used for treating hypoxemia in a subject having respiratory failure. Thus, the pharmaceutical composition of the present invention for intraintestinal administration (preferably for administration into a large intestine, more preferably for intrarectal administration) can be for use in supplying oxygen to the blood of a subject.

The present invention provides, as a further aspect, a perfluorocarbon dissolving oxygen therein for intraintestinal administration (preferably for administration into a large intestine, more preferably for intrarectal administration). The perfluorocarbon of the present invention dissolving oxygen therein can be used for treating hypoxemia. The perfluorocarbon of the present invention dissolving oxygen therein can be used for treating respiratory failure in a subject. The perfluorocarbon of the present invention dissolving oxygen therein can be used for treating hypoxemia in a subject having respiratory failure. Thus, the perfluorocarbon of the present invention dissolving oxygen therein can be for use in supplying oxygen to the blood of a subject.

The present invention provides, as a further aspect, use of oxygen gas in manufacture of a pharmaceutical composition in the form of gas for intraintestinal administration (preferably for administration into a large intestine, more preferably for intrarectal administration). The pharmaceutical composition of the present invention in the form of gas for intraintestinal administration (preferably for administration into a large intestine, more preferably for intrarectal administration) can be used for treating hypoxemia. The pharmaceutical composition of the present invention in the form of gas for intraintestinal administration (preferably for administration into a large intestine, more preferably for intrarectal administration) can be used for treating respiratory failure in a subject. The pharmaceutical composition of the present invention in the form of gas for intraintestinal administration (preferably for administration into a large intestine, more preferably for intrarectal administration) can be used for treating hypoxemia in a subject having respiratory failure. The pharmaceutical composition of the present invention in the form of gas for intraintestinal administration (preferably for administration into a large intestine, more preferably for intrarectal administration) can be used for decreasing the blood carbon dioxide partial pressure of a subject. Thus, the pharmaceutical composition of the present invention in the form of gas for intraintestinal administration (preferably for administration into a large intestine, more preferably for intrarectal administration) can be for use in supplying oxygen to the blood of a subject.

The present invention provides, as a further aspect, oxygen gas for intraintestinal administration (preferably for administration into a large intestine, more preferably for intrarectal administration). The oxygen gas of the present invention can be used for treating hypoxemia. The oxygen gas of the present invention can be used for treating respiratory failure in a subject. The oxygen gas of the present invention can be used for treating hypoxemia in a subject having respiratory failure. Thus, the oxygen gas of the present invention can be for use in supplying oxygen to the blood of a subject.

The pharmaceutical compositions of the first aspect and second aspect of the present invention can be each administered to a subject to keep the blood oxygen partial pressure of the subject at 60 mmHg or higher. The pharmaceutical compositions of the first aspect and second aspect of the present invention can be each administered to a subject once per day or through multiple administration. The pharmaceutical compositions of the first aspect and second aspect of the present invention can be each used in combination with artificial ventilation using a ventilator and an oxygenation (e.g., an extracorporeal membrane oxygenation).

Examples

### [Method]

### Animals

C57BL/6J mice were purchased from Japan SLC, Inc. (Shizuoka, Japan). These mice were grown in a pathogen-free environment with free access to water and foods under 12-hour light-and-dark cycles. All the animal growth and experiments conducted were in accordance with guidelines by the relevant organization and Japan (Ministry of Education, Culture, Sports, Science and Technology), and had been approved by the Animal Research Committee, Kyoto University (approval number: Med Kyo 19583).

### Ventilation of Intestinal Gas

An intestinal gas ventilation (IGV) system for direct administration of pure oxygen to the large intestine was designed. First, initial evaluation was performed for six intestinal tract scraping methods (see Table 1). Mice were randomly assigned to eight groups (Table 1): group 1 - sham (n = 3); group 2 - without scraping (n = 3); group 3 - oral administration of 2% dextran sulfate sodium (DSS) for 5 days (n = 5); group 4 - intestinal administration of 2% DSS for 5 days (n = 5); group 5 - oral administration of 2% DSS for 5 days followed by infusion of basic fibroblast growth factor (bFGF) once per intestinal tract (n = 6); group 6 - mild mechanical mucosal abrasion (n = 10); group 7 - moderate mechanical mucosal abrasion (n = 10); and group 8 - intense mechanical mucosal abrasion (n = 5). Group 1 to group 8 were subjected to skin incision under anesthesia with ketamine (80 to 100 mg/kg) and xylazine (10 mg/kg), and blood was collected. Pure oxygen was administered to each large intestine in a range of 4 cm in the intestine, and oxygen levels in the inferior vena cava were estimated for groups 2 to 8. Group 1 was analyzed for venous gas, and group 5 was analyzed for venous gas 3 days after injection of bFGF. For mechanical groups, scraping of the intestinal mucosa was performed under anesthesia (see Table 1), by inducing mechanical brushing with use of an interdental brush over the whole length of the intestine for a predetermined time. For group 9 to group 11, artificial ventilation (ventilation: 500 µL/cycle, respiration rate: 100 to 120 respiration/min, PEEP: 2 cm H₂O) was performed. Mice were randomly assigned to three groups. Specifically, mice were randomly assigned to the following three groups: group 9 - sham group (n = 10); group 10 - scraping-less intestinal gas ventilation group (scraping-less IGV group, n = 7); and group 11 - mucosal-scraping-involving intestinal gas ventilation group (moderate scraping-involving IGV group, n = 11).

### [Table 2]

**Table 2: List of groups**

| | | IGV | ILV | Mucosal abrasion | Additional conditions | FiO₂ | Airway ventilation | Evaluation | Figure number |
|---|---|---|---|---|---|---|---|---|---|
| IGV | Group 1 (Sham) | - | - | - | - | 0.21 | - | venous oxygenation (IVC), histopathology | 1C, 4A |
| | Group 2 | + | - | - | - | 0.21 | - | venous oxygenation (IVC) | 4A |
| | Group 3 | + | - | chemical | Oral DSS | 0.21 | - | venous oxygenation (IVC) | 4A |
| | Group 4 | + | - | chemical | Intrarectal DSS | 0.21 | - | venous oxygenation (IVC) | 4A |
| | Group 5 | + | - | chemical | Oral DSS+bFGF | 0.21 | - | venous oxygenation (IVC) | 4A |
| | Group 6 | + | - | mechanical | mild mucosal abrasion | 0.21 | - | venous oxygenation (IVC) | 4A |
| | Group 7 | + | - | mechanical | moderate mucosal abrasion | 0.21 | - | venous oxygenation (IVC), histopathology | 1C, 4A, 48 |
| | Group 8 | + | - | mechanical | intense mucosal abrasion | 0.21 | - | venous oxygenation (IVC) | 4A |
| | Group 9 (Sham) | - | - | - | - | 0.21 | + | venous oxygenation (IVC) | 2B |
| | Group 10 | + | - | - | - | 0.21 | + | venous oxygenation (IVC) | 2B |
| | Group 11 | + | - | mechanical | mild mucosal abrasion | 0.21 | + | venous oxygenation (IVC) | 2B |
| ILV | Group 12 | - | + | - | PFC (120 min) | 0.21 | + | venous oxygenation (IVC) | 3C |
| Respiratory failure model | Group 13 | - | - | - | - | 0.1 | + | arterial oxygenation (left ventricle) | 2C |
| | Group 14 | + | - | mechanical | mild mucosal abrasion | 0.1 | + | arterial oxygenation (left ventricle) | 2C |
| | Group 15 | - | - | - | - | 0.08 | + | survival rate | 2A |
| | Group 16 | + | - | - | - | 0.08 | + | survival rate | 2A |
| | Group 17 | + | - | mechanical | mild mucosal abrasion | 0.08 | + | survival rate | 2A |
| | Group 18 | - | + | - | PFC (60 min) | 0.1 | + | arterial oxygenation (left ventricle) | 3D |
| | Group 19 | - | + | - | PFC (120 min) | 0.1 | + | arterial oxygenation (left ventricle) | 3D |

### Intestinal Gas Ventilation (Intestinal Fluid Ventilation)

With use of a liquid perfluorocarbon (PFC)¹²⁻¹⁶, an intestinal gas ventilation (ILV) system was developed. Mice were randomly assigned to two groups: sham group (group 9, n = 10); and intestinal gas ventilation group (group 12, n = 12). For the intestinal gas ventilation (ILV), a PFC (octadecafluorodecahydronaphthalene, Wako Pure Chemical Industries, Ltd., Osaka, Japan) was bubbled with pure oxygen for 45 minutes to dissolve oxygen therein. The PFC in a volume of 1 mL was administered to each mouse via the intestinal tract, and 120 minutes thereafter the oxygen partial pressure in the inferior vena cava at the level of the right renal vein was analyzed.

### Recovery of Respiratory Failure by IGV and ILV

In IGV experiment, animals were randomly assigned to two groups (Table 1): group 13 - intestinal-ventilation-less group (sham group, n = 4); and group 14 - intestinal gas ventilation group with scraping of mucosa (moderate scraping, IGV group, n = 4). The skin and tracheal tube were incised under anesthesia, and mechanical ventilation was performed as described above. For group 14, pure oxygen was administered into the intestinal lumen. Ten minutes after artificial ventilation, hypoxic gas (FiO₂ 0.10) was administered to the airway of each mouse, and the oxygen partial pressure in the left ventricle of the heart 10 minutes after the administration of hypoxic gas was estimated. Survival experiment was carried out under conditions with FiO₂ 0.08 because FiO₂ 0.10 is nonlethal. Animals were randomly assigned to three groups (Table 1). Specifically, animals were randomly assigned to the following three groups: group 15 - sham group (n = 3); group 16 - intestinal gas ventilation group (IGV without mucosal abrasion, n = 3); and group 17 - mucosal-abrasion-involving intestinal gas ventilation group (IGV with moderate mucosal abrasion, n = 4). Survival rates were compared across groups 15, 16, and 17.

In ILV experiment, mice were randomly assigned to two groups: sham group (group 13, n = 4); and ILV groups (group 18, n = 4; group 19, n = 4). The PFC was given to each mouse via the intestinal tract in the above-described manner, and 60 minutes and 120 minutes thereafter the oxygen partial pressure in the left ventricle of the heart under inhalation of hypoxic gas was analyzed (FiO₂ 0.10, inhalation time: 10 minutes).

### Behavior Analysis

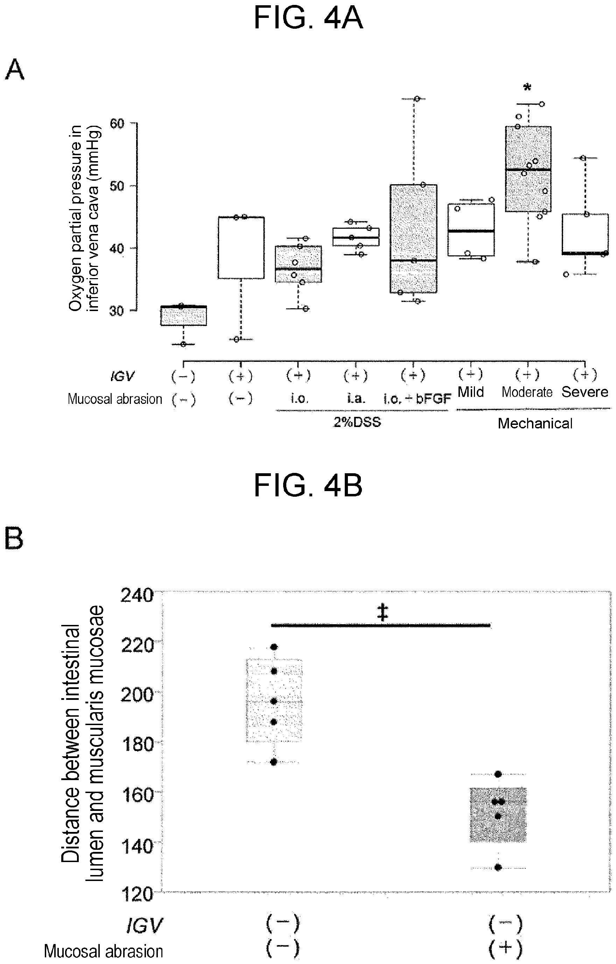

Behavioral improvement in ILV-treated hypoxic mice was analyzed. The mobilities of mice were monitored for a set of two parameters from a fitted ellipse (centroidal positions (x, y)). With use of these parameters, walking distance was calculated every 20 seconds for mice of a control group and a treated group (both n = 3). The walking behavior of each mouse was observed for 5 minutes in total. For the treated group, an O₂-labeled perfluorocarbon was used as described above, and inhalation of hypoxic gas (FiO₂ 0.10) was performed 120 minutes after the injection to the intestinal tract (Figure 4A).

### Hematoxylin-Eosin Staining

Adults of the loach *Misgurnus anguillicaudatus* (average body length: approximately 15 cm) were purchased from Meito-Suien K.K. (Aichi, Japan). The intestines of C57BL/6J mice and adult loaches were analyzed. The intestines of the mice were grouped into three groups: intestinal-respiration-less group (group 1, sham group); intestinal respiration group (group 2, mucosal-abrasion-less IGV group); and intestinal respiration group subjected to mucosal abrasion (group 7, mucosal-abrasion-involving IGV group). The loaches were subjected to histopathological analysis of the posterior intestine, which has been reported to perform intestinal respiration.

### Histological Quantification

Slides prepared from formalin-fixed paraffin-embedded tissues were mounted and then stained with hematoxylin and eosin. Under a high-power microscope (400×, optical microscope), 20 fields were randomly selected, and blindly scored for different parameters. For slides from samples of group 7, the distance between the intestinal lumen and the muscular mucosa was estimated at five random positions. Slides were scored by three independent raters including an experienced pathologist and surgeon.

### Immunochemical staining with Hypoxyprobe^{™}

Immunochemical analysis for intestinal tract regions to be subjected to intestinal respiration was carried out by using a Hypoxyprobe^{™}-1 Kit (HP2-100, Hypoxyprobe, Burlington, Massachusetts) in accordance with an instruction provided by the manufacturer¹⁷. Briefly speaking, a solution of Hypoxyprobe^{™}-1 was infused into the peritoneum of each mouse at a dose of 60 mg/kg body weight. A section of the intestine was fixed with 100 neutral buffered formalin, subjected to antigen retrieval (blocking solution, at 90°C for 20 minutes), washed with PBST (PBS + 0.1% Triton X-100), and exposed to a FITC-labeled anti-pimonidazole mouse monoclonal primary antibody at room temperature for 60 minutes. After additional washing with PBST, a peroxidase-labeled anti-FITC rabbit secondary antibody was applied, and the resultant was visualized under the optical microscope BX 43 (Olympus Corporation, Tokyo, Japan). As shown, the signal intensities from 10 mucosal epithelial cells adjacent to each villus of the intestinal tract were each scored as a negative cell (0 points), a weakly positive cell (1 point), or strongly positive cell (2 points). Evaluation was performed for five items in total, and the total score was calculated. Slides were independently rated by three raters including an experienced pathologist and surgeon.

### RT-qPCR Analysis

To evaluate molecular change relating to moderate and intense mechanical mucosal abrasion in the intestine, mice were sacrificed 0 minutes, 20 minutes, 6 hours, and 24 hours after mucosal abrasion for the intestinal mucosa (n = 12 for each mechanical mucosal abrasion). Intestinal tract tissues were collected, and RNA was then immediately preserved (QIAGEN, Hilden, Germany). Total RNA from each tissue was extracted by using a FastGene RNA Basic Kit (NIPPON Genetics Co., Ltd., Tokyo, Japan). Single-stranded cDNA was synthesized by using ReverTra Ace qPCR Master Mix with gDNA Remover (TOYOBO CO., LTD., Osaka, Japan). Quantitative PCR was performed by using THUNDERBIRD SYBR qPCR Mix (TOYOBO CO., LTD., Osaka, Japan) on a QuantStudio 3 real time PCR system (Thermo Fisher Scientific, Tokyo, Japan). For relative quantification, a standard curve was prepared for each gene, and the expression level of each gene was normalized to an *Rn28s* gene. The specific primers used are as follows.
*Vegfa*-F (SEQ ID NO: 1) 5'-AGGCTGCTGTAACGATGAAG-3' and
*Vegfa*-R (SEQ ID NO: 2) 5'-TCTCCTATGTGCTGGCTTTG-3';
*Anxa1*-F (SEQ ID NO: 3) 5'- CCAGCACTCCAGCTTTCTTT-3' and
*Anxa1*-R (SEQ ID NO: 4) 5'- TCCGAACGGGAGACCATAAT-3';
*Spon1*-F (SEQ ID NO: 5) 5'- AGAGAACCAGGAGGGAGATAAG-3' and
*Spon1*-R (SEQ ID NO: 6) 5'- GCCACAGGACAGTTACTCATAAA-3';
*Glud1*-F (SEQ ID NO: 7) 5'- TACCGTTTGGAGGTGCTAAAG-3' and
*Glud1*-R (SEQ ID NO: 8) 5'- CCATAGTGAACCTCCGTGTAAT-3'

### Statistical Analysis for Results

Statistical analysis was performed by using JMP-Pro 14 software (Cary, North Carolina, U.S.). Survival rates were estimated by using the Kaplan-Meier method, and intergroup differences were tested by using log-rank test. Differences in intravascular oxygen partial pressure among the sham group, the mucosal-abrasion-less intestinal gas ventilation group, and the mucosal-abrasion-involving intestinal gas ventilation group were analyzed by Wilcoxon signed-rank sum test. p values lower than 0.05 were considered to be statistically significant. All data are represented as average ± standard deviation (SD).

### [Results]

### Intestinal Gas Ventilation (IGV) Alleviates Local Hypoxic Disorder.

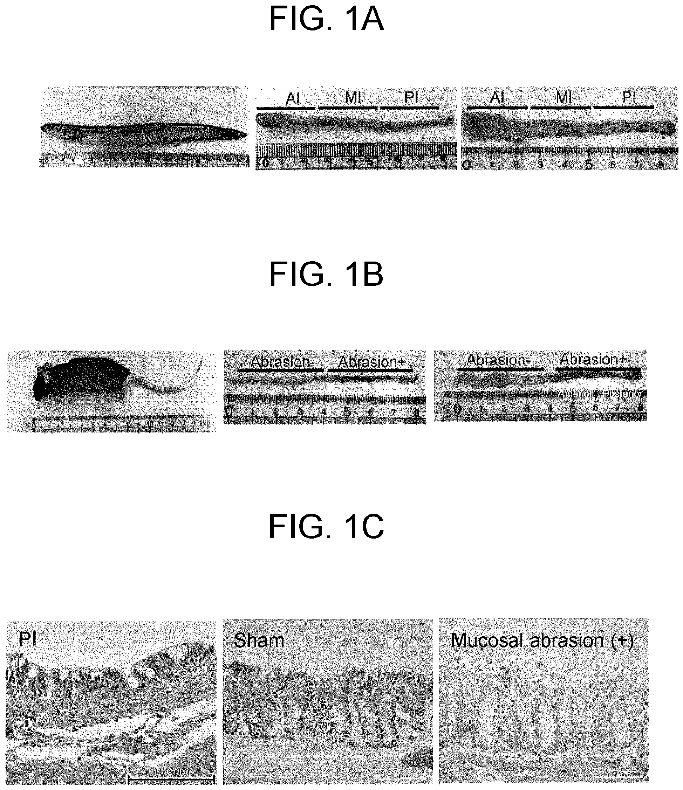

Histopathological staining analysis has demonstrated that the posterior intestine of loaches has plenty of microvessels and erythrocytes, which are believed to be important for the ability of intestinal respiration¹⁸. We hypothesized that respiration is more improved as the intestinal lumen is closer to microvessels, and some mouse models were selected to test the hypothesis. To allow more efficient intraluminal access to submucosal vessels, we applied various chemical and mechanical mucosal abrasion protocols to remove the epithelium lining the distal intestine (Table 1, groups 1 to 8, Figures 1A and 1B). Among them, an appropriate mechanical mucosal abrasion protocol gave a dissolved oxygen concentration 13.6 ± 5.66 mmHg higher than in mucosal-abrasion-less condition, being the most efficient for enabling oxygen exchange with the intestinal lumen (Figure 4A). The distance between the intestinal lumen and the muscular mucosa in the mucosal-abrasion-involving IGV group was significantly shorter than that in the sham group (IGV with mucosal abrasion: 151.7 ± 13.7 µm, sham: 196 ± 17.8 µm, p = 0.012, Figure 4B). Importantly, mortality was 0% for all of the following groups: the sham group; the group not subjected to mucosal abrasion; the 2% DSS oral administration group; and the moderate mechanical mucosal abrasion group, which demonstrated that those methods are nonlethal. For the other groups, mucosal abrasion for the intestinal tract resulted in 16 to 20% of mortalities due to colitis or intestinal hemorrhage. Those histopathological findings suggest similarity between the posterior intestine of loaches and the distal intestine (i.e., the rectum) subjected to mucosal abrasion in mice of group 7 (Figure 1C).

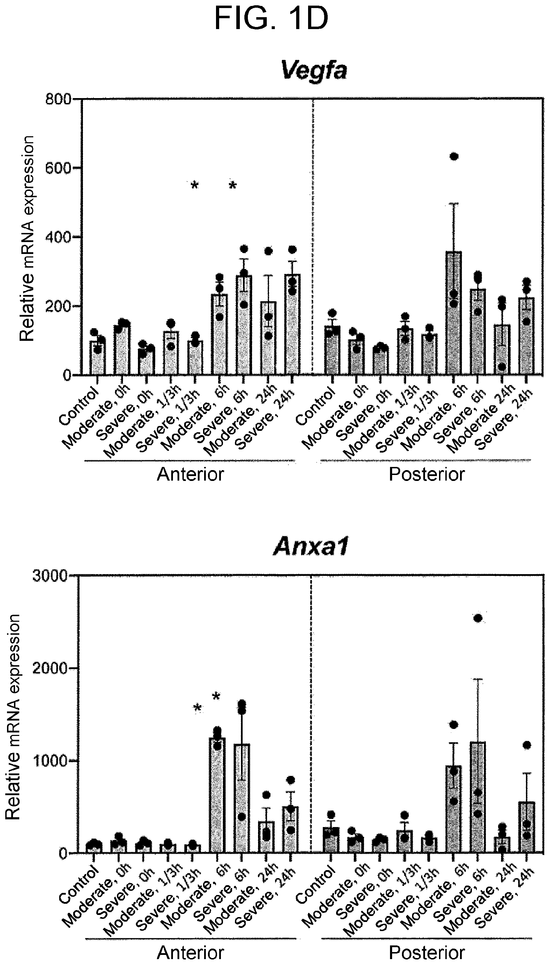

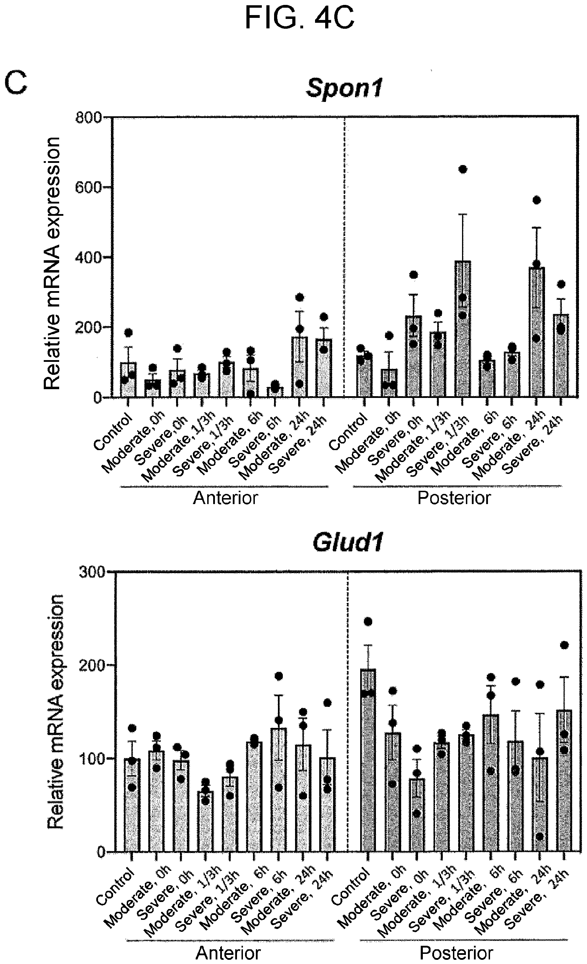

*M. anguillicaudatus* begins to develop the function of air breathing in the intestine about 10 days after hatching. Recent RNA-seq analysis of the posterior intestine of *M. anguillicaudatus* at different stages of development has revealed the presence of gene signatures closely related to acquisition of the function of intestinal respiratory such as upregulation of vascularization (VEGFA, SPON1B) and mucosal inflammation (ANXA1) and downregulation of oxidative phosphorylation (GDH)¹⁸. Focusing on *Vegfa, Spon1*, *Anxa1*, and *Glud1*, which are the corresponding mouse homologues, we examined whether mechanical mucosal abrasion for the lumen of the intestinal tract induces similar change in gene expression in the distal intestine of mice. Quantitative RT-PCR analysis showed that *Vegfa* and *Anxa1* exhibited significant increase 6 hours and 24 hours after moderate or intense mucosal abrasion, in particular, in the anterior region of the distal intestine dissected, whereas *Spon1* and *Glud1* exhibited only slight change as compared with the control (without mucosal abrasion) (Figure 1D and Figure 4C)¹⁸.

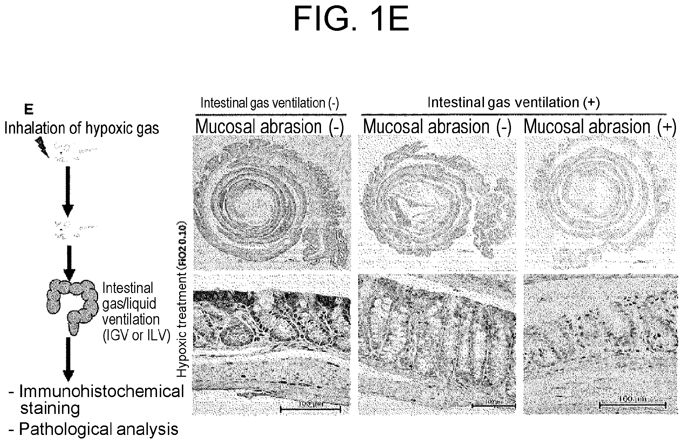

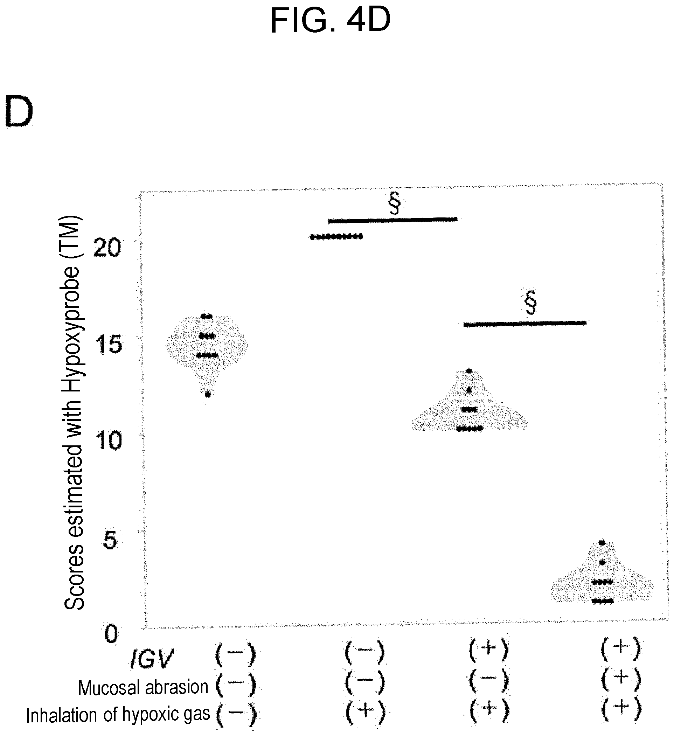

To further evaluate physiological change due to hypoxic condition in local tissue, immunochemical staining of the intestine was performed for mice (with or without IGV) subjected to hypoxic treatment (FiO₂ 0.10) with use of a Hypoxyprobe^{™}-1 kit. While positive stained cells (hypoxic cells) in sham mice were limited to the mucosal epithelium in the absence of IGV and hypoxic condition, the number of strongly positive cells increased after inhalation of hypoxic gas, and the cells were distributed in the mucosa, beneath the mucosa, and in the connective tissue (Figure 1E). In the mucosal-abrasion-less IGV group and the mucosal-abrasion-involving IGV group, by contrast, the number of hypoxic cells found in hypoxic regions was far smaller (Figure 1E). Pathological scoring showed that the scores in the mucosal-abrasion-less IGV group and the mucosal-abrasion-involving IGV group were significantly lower than that of the IGV-less group under inhalation of hypoxic gas (IGV with mucosal abrasion vs. IGV without mucosal abrasion; p = 0.0001, IGV without mucosal abrasion vs. sham; p < 0.0001), demonstrating that intestinal gas ventilation significantly alleviates local hypoxic impairment (Figure 1E and Figure 4D).

### Systemic Oxygenation by Intestinal Gas Ventilation

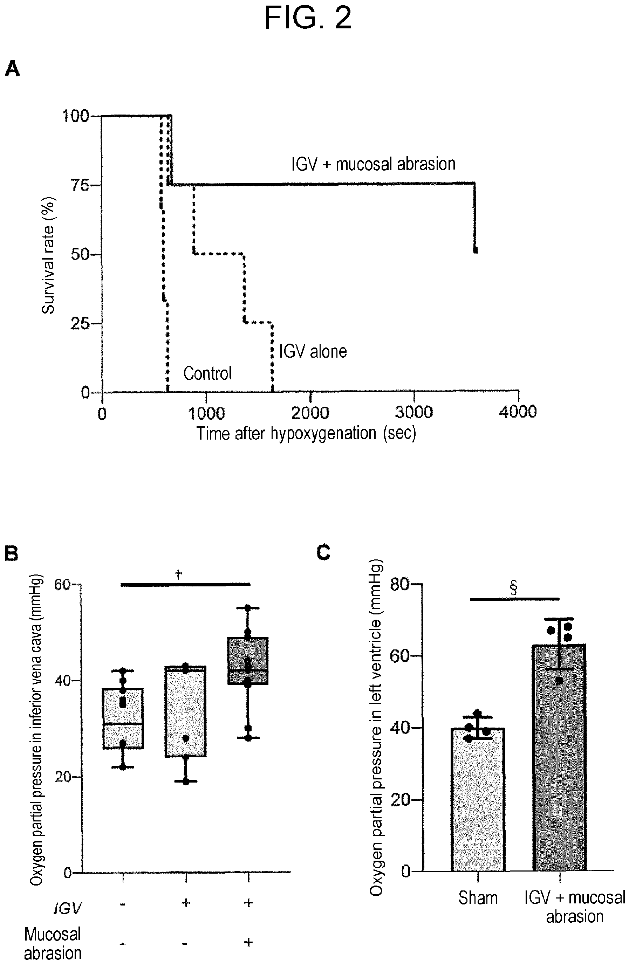

Next, assessment was performed to determine whether IGV has systemic oxygenation effect. First, the benefit of IGV to survival rates under lethal hypoxic condition (FiO₂ 0.08, Table 1, groups 15 to 17) was evaluated. Before 3000 seconds (50 minutes) elapsed after inhalation of hypoxic gas, the survival rates of the control group and the group not subjected to mucosal abrasion had reached 0%. Surprisingly, the survival rate of the mucosal-abrasion-involving IGV group after 50 minutes was 75%, which was statistically significant as compared with the control group (IGV with mucosal abrasion, 3/4; IGV without mucosal abrasion, 0/3; sham group, 0/3; P < 0.001, Kaplan-Meier method and log-rank test, Figure 2A).

The oxygen partial pressure in the inferior vena cava in the mucosal-abrasion-involving IGV group was statistically higher than that in the sham group (p = 0.004). The oxygen partial pressures in the sham group, the mucosal-abrasion-less IGV group, and the mucosal-abrasion-involving IGV group were 31.6 ± 7.44, 32.9 ± 10.6, and 40.3 ± 9.57 mmHg, respectively (FiO₂ 0.21, Figure 2B, groups 9 to 11). Similarly, the arterial oxygen partial pressure in the left ventricle of the heart in inhalation of hypoxic gas in the mucosal-abrasion-involving IGV group was statistically higher than that in the sham group (p = 0.030). The oxygen pressure levels in the sham group and the mucosal-abrasion-involving IGV group were 40.0 ± 2.94 mmHg and 63.3 ± 6.94 mmHg, respectively (FiO₂ 0.10, Figure 2C, groups 13 and 14). These results revealed that intestinal gas ventilation is effective for alleviating lethal hypoxic condition in the presence of mucosal abrasion for the mucosa of the distal intestine.

### Therapeutic Effect of Intestinal Gas Ventilation

Oxygenated perfluorocarbon (PFC) liquid is a substance developed for improving oxygenation, and also known as Liquid Ventilation (LV). For its biocompatibility with humans, intrapulmonary application of PFC in liquid or aerosol has been already used in clinical situations to mitigate pulmonary injury in the case of severe respiratory failure. Because the particle size of PFC is inferred to give tissue permeability (having a diameter smaller than several micrometers)¹⁹, we examined use of PFC, as an inert carrier of oxygen in an intestinal delivery method, with the expectation that PFC provides more advantages than the approach based on mechanical mucosal abrasion for the distal intestine.

First, an O₂-containing PFC was prepared (by O₂ bubbling at 1 L/min over 45 minutes; Figure 3A). For mice (groups 12, 18, 19; hereinafter, referred to as intestinal gas ventilation (ILV) groups), the liquid of PFC was then infused into the intestinal tract (i.e., the rectum) in a total volume of 1 mL/mouse. The average oxygen pressure in the PFC after O₂ bubbling for 45 minutes was 438 ± 19.9 mmHg (n = 3, Figure 4E). Effects on the treated mice were evaluated by mobility assay. In summary, the walking distances of the mice were calculated to find statistical increase for the group with O₂-PFC liquid infusion therapy after inhalation of hypoxic gas (the ILV group with administration of the O₂-containing PFC) as compared with the control group (FiO₂ 0.10, control group, 0.408 ± 1.02 cm per 20 seconds; FiO₂ 0.10, treated group, 3.34 ± 4.05 cm, p < 0.0001, Figure 3B) .

As an experiment for reproduction, LEWIS rats (male) ranging from 250 to 350 g were grouped into three groups: control group; I-EVA group (using research-grade perfluorodecalin (PFD) from Wako Pure Chemical Industries, Ltd.); and I-EVA group (using clinical-grade perfluorodecalin from F2C). Perfluorodecalin bubbled with pure oxygen gas was administered into the rectum of each rat at 20 mL/kg, and 15 minutes and 120 minutes thereafter the arterial blood was collected under anesthesia and 21% oxygen inhalation, and the oxygen partial pressure was measured.

Next, C57BL6J mice ranging from 20 to 30 g were grouped into two group: control group (physiological saline administration group); and I-EVA group (using perfluorodecalin from F2C with oxygenation). The mice of the control group were each anesthetized by intraperitoneal administration of ketamine (80 to 100 mg/kg) and xylazine (10 mg/kg) and subjected to inhalation under 15% hypoxic condition, and, when SpO₂ just exceeded 70%, oxygenated perfluorodecalin was administered into the rectum at 40 mL/kg, and effects on respiratory failure were validated by using an SpO₂ monitor. After the completion of the experiment, the perfluorodecalin enterally administered was recovered, and the carbon dioxide concentration was measured to compare with that measured before the operation.

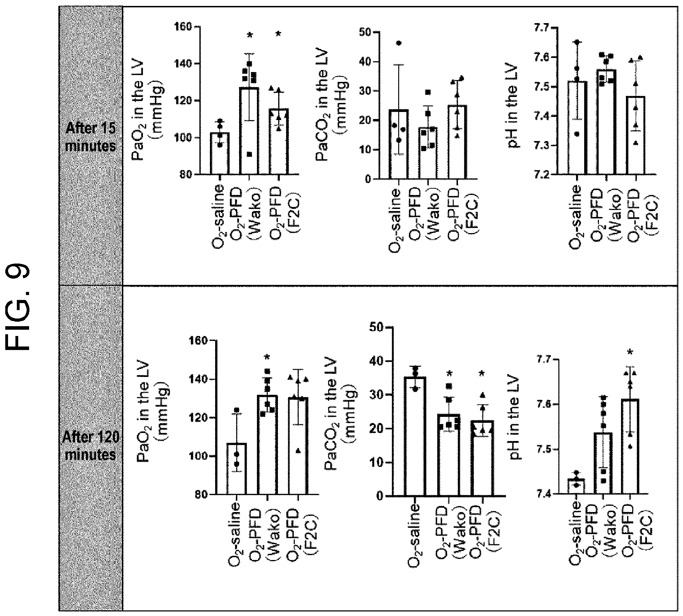

The results were as shown in Figure 9. As seen from Figure 9, an effect of enhancing blood oxygen partial pressure was found both 15 minutes and 120 minutes after administration for the group with administration of the oxygenated PFC as compared with the group with administration of physiological saline. In addition, as demonstrated in Figure 9, an effect of significantly decreasing blood carbon dioxide partial pressure was found 120 minutes after administration for the group with administration of the oxygenated PFC as compared with the group with administration of physiological saline. These results suggest that PFC not only has abilities to dissolve oxygen therein and supply the oxygen into blood, but also is capable of decreasing blood carbon dioxide concentration by adsorbing carbon dioxide in blood.

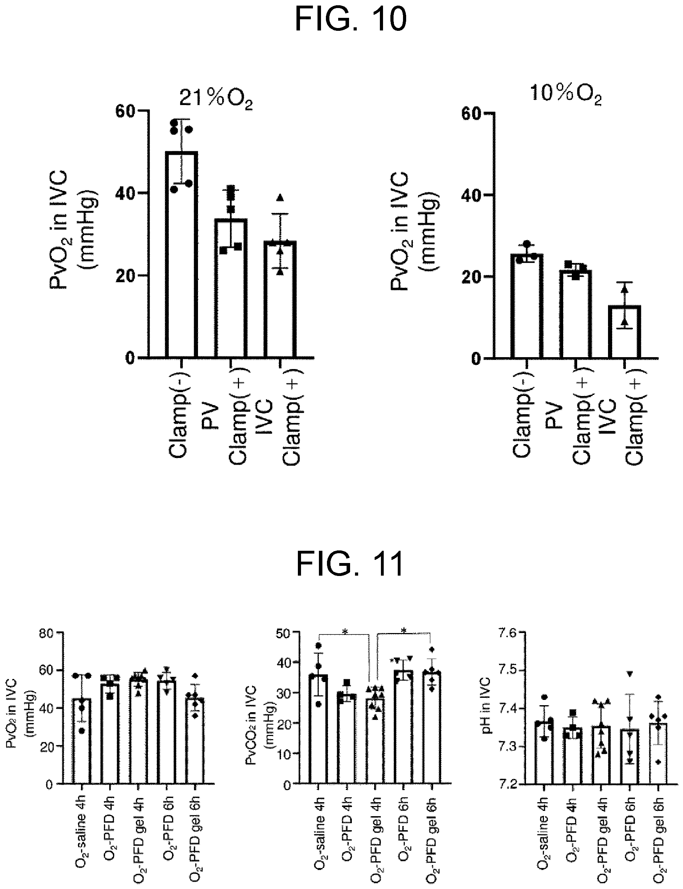

Next, to investigate vessels involved in enteral respiration, vascular clamping (Clamp) was performed for the inferior vena cava (IVC) or the portal vein (PV). Specifically, C57BL6J mice (male) ranging from 20 to 30 g were grouped into three groups: control group; portal vein clamp group; and vena cava clamp group. Further, the mice of each group were divided into mice under room air (under 21% oxygen) and mice under hypoxic condition (10% oxygen). For the mice under 21% oxygen inhalation, perfluorodecalin, from Wako Pure Chemical Industries, Ltd., bubbled with pure oxygen was administered into the rectum of each mouse under anesthesia at 40 mL/kg, blood was collected from the vena cava 10 minutes after administration, and the oxygen partial pressure was measured. For the mice under 10% oxygen inhalation, perfluorodecalin bubbled with pure oxygen was administered into the rectum of each mouse under anesthesia at 40 mL/kg, blood was collected from the vena cava 20 minutes after administration, and 10 minutes after inhalation of hypoxic gas, and the oxygen partial pressure was measured. The results were as shown in Figure 10. As seen from Figure 10, decreased venous cava oxygen partial pressure was given by clamping for any of the PV and the IVC. These results suggested the involvement of multiple vessels, not only one vessel.

Further, gelling of oxygenated PFC was attempted for easier handling of PFC. Perfluorodecalin from Wako Pure Chemical Industries, Ltd. was used as the PFC, and gelling of perfluorodecalin was attempted by mixing with Spring Powder WO from AKATAZEN Co., Ltd. (head office: Osaka, Japan). Stock solution of perfluorodecalin was prepared, and the stock solution alone, a mixture obtained by dissolving the stock solution in physiological saline at stock soluion:physiological saline = 1 mL:1 mL, and that at stock soluion:physiological saline = 1 mL:3 mL were each further mixed with Spring Powder WO, and it was revealed that the 1:1 mixture had a viscosity suitable for rectal administration (Spring Powder WO: 0.1 g). The components of Spring Powder WO are sucrose fatty acid ester (65%, emulsifying agent), carrageenan (14%, gelling agent), carob bean gum (9.1%, gelling agent), xanthan gum (7.7%, gelling agent), potassium chloride (2.63%, auxiliary agent), and food materials (1.57%, food materials). Blood was collected form the vena cava 4 hours and 6 hours after administration, and the oxygen partial pressure was measured. The results were as shown in Figure 11. As demonstrated in Figure 11, enteral administration of the gelled PFC gave enhanced venous blood oxygen partial pressure. As seen from Figure 11, enteral administration of the gelled PFC gave decreased venous carbon dioxide partial pressure. These results demonstrated that PFC may be gelled as necessary.

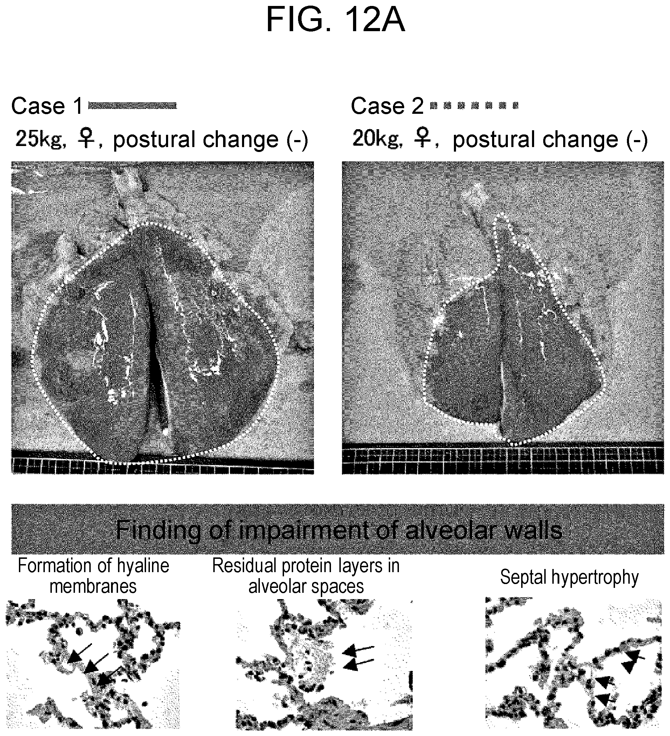

Furthermore, an acute respiratory distress syndrome (ARDS) pig model was produced, and improvement of oxygen partial pressure by intrarectal administration of oxygenated PFC was examined. First, micromini pigs and pork pigs (female) were used to produce an ARDS model. Physiological saline was directly administered into the airway at 40 mL/kg. The lungs of the pigs were checked and, as shown in Figure 12A, found to have been impaired in the regions surrounded by dotted lines, each region occupying a large part of the lung. Histological examination of lung tissue confirmed impairment of alveolar walls such as the formation of hyaline membranes, the formation of residual protein layers in alveolar spaces, and septal hypertrophy. In addition, the PaO₂/FiO₂ ratio was 100 or lower, and these symptoms suggested that the pigs underwent the onset of moderate to severe ARDS.

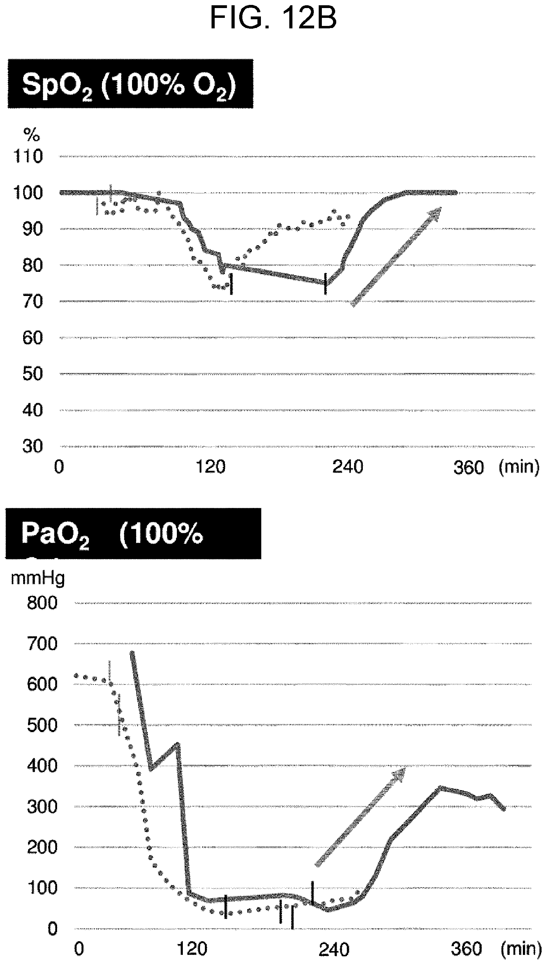

Then, the thus-produced ARDS pigs were each subjected to laparotomy by median incision, and a drain for administration of perfluorodecalin was placed at a depth of 40 cm from the anus, and an Argyle^{™} Dennis^{™} Colorectal Tube for recovery of perfluorodecalin was placed in the anus side. To each pig of the ARDS model, perfluorodecalin from F2C after being oxygenated was repeatedly administered at 20 mL/kg, the arterial blood oxygen saturation, arterial blood oxygen partial pressure, and venous blood oxygen partial pressure were measured, and, after the completion of the experiment, the intestinal tract, spleen, liver, etc., of each pig were histopathologically evaluated for the presence or absence of any adverse event. The results were as shown in Figure 12B.

As seen from Figure 12B, the arterial blood oxygen saturation (SpO₂) was improved by 20 to 25% through the administration. The oxygen partial pressure was improved by about 300 mmHg. These results demonstrated the efficacy of intrarectal administration of oxygenated PFC to pigs having an impaired lung and the resulting respiratory failure. The pigs did not exhibit marked acidosis. Additionally, intrarectal administration of the oxygenated PFC to hypoxic pigs subjected to hypoxic condition improved the oxygen saturation, similarly, and induced improvement in blood oxygen partial pressure and decrease in carbon dioxide partial pressure.

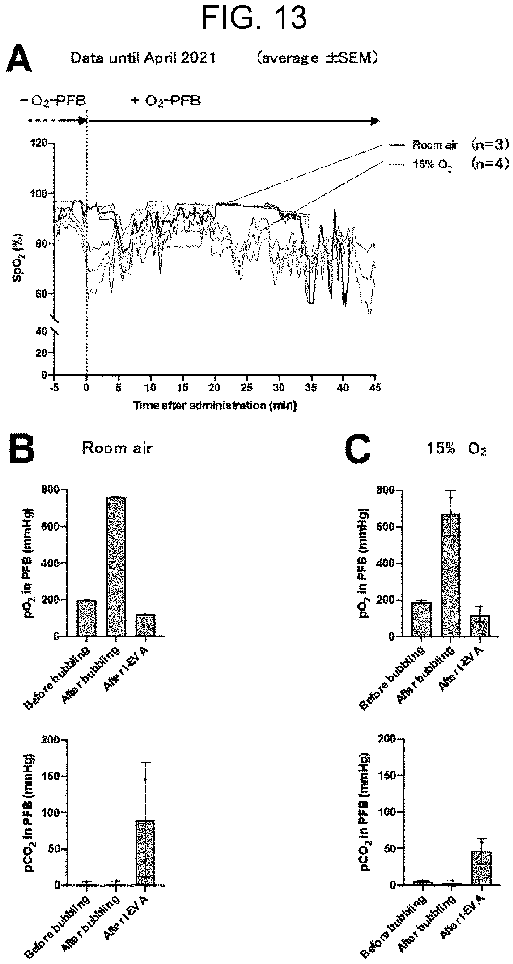

Experiment was carried out to administer oxygenated perflubron to mice having respiratory failure. PFB manufactured by OriGen Biomedical (Perflubron, product name: Liquivent) was oxygenated by oxygen bubbling. Male C57BL6J mice (male) ranging from 20 to 30 g were grouped into two groups: control group (physiological saline administration group); and I-EVA group (using oxygenated perflubron). The mice of the control group were each anesthetized by intraperitoneal administration of ketamine (80 to 100 mg/kg) and xylazine (10 mg/kg) and subjected to inhalation under 15% hypoxic condition, and, when SpO₂ just exceeded 70%, perflubron was administered into the rectum at 40 mL/kg, and effects on respiratory failure were validated by using an SpO₂ monitor. The results were as shown in Figure 13.

As seen from Figure 13, the mouse groups underwent large reduction in SpO₂ immediately after inhalation of hypoxic gas was initiated, but exhibited significant recovery of SpO₂ due to intrarectal administration of the oxygenated PFB (panel A of Figure 13). The oxygen partial pressures of the PFB before oxygen bubbling, after the bubbling, and after intrarectal administration were measured to find that the PFB was provided with enhanced oxygen partial pressure by oxygen bubbling (panels B and C of Figure 13). This result indicates that the PFB has an ability to be preferably oxygenated. The PFB underwent decrease in the oxygen partial pressure after intrarectal administration (panels B and C of Figure 13). This result indicates that the PFB released oxygen in the intestine after administration, and at least part of the released oxygen was supplied into blood. The carbon dioxide partial pressures of the PFB before oxygen bubbling, after the bubbling, and after intrarectal administration were measured to find that the carbon dioxide partial pressure increased after intrarectal administration (panels B and C of Figure 13). This result indicates that in the rectum the PFB adsorbed carbon dioxide contained in blood. Thus, the PFB had an ability to supply oxygen to blood through intrarectal administration and allow carbon dioxide to be discharged from blood.

The blood PDF concentration 0.5 hours, 2 hours, and 24 hours after administration of perfluorodecalin (PFD) was measured by GC/MS/MS at times (n = 6). The result showed that the blood PDF concentration was below the detection limit (1 µg/mL) at any of times 0.5 hours, 2 hours, and 24 hours after administration. Systemic toxicity after six cycles of administration of PDF was examined. Analysis of serum gave results as shown in Table 2 in the following.

[Table 3]

**Table 3 Results of analysis of serum after administration of PFD**

| | | | | P value |
|---|---|---|---|---|
| Substance | Saline | PFD | PFD | |
| Dose volume(ml/head) | 1 | 1 | 1.5 | |
| Number of doses (time/day) | 6 | 6 | 6 | |

| | | | | |
|---|---|---|---|---|
| Erythrocytes (× 10⁴/ *µ*l) | 778 ± 40 | 752 ± 24 | 791 ± 24 | N.S. |
| Hematocrit (%) | 45.7 ± 2.4 | 45.0 ± 1.1 | 45.6 ± 1.4 | N.S. |
| Hemoglobin (g/dL) | 15.3 ± 0.8 | 15.1 ± 0.7 | 15.2 ± 0.6 | N.S. |
| Platelets (10⁴/ *µ*L) | 103 ± 16.1 | 112 ± 11.5 | 123.5 ± 20.1 | N.S. |
| Leukocytes (10²/ *µ*L) | 82 ± 10 | 102 ± 18 | 88 ± 5 | N.S. |
| Albumin (g/dL) | 2.6 ± 0.1 | 2.6 ± 0.1 | 2.6 ± 0.1 | N.S. |
| Glucose (mg/dL) | 124 ± 7 | 123 ± 22 | 123 ± 18 | N.S. |
| Total cholesterol (mg/dL) | 62 ± 10 | 75 ± 20 | 71 ± 22 | N.S. |
| Tri-glyceride (mg/dL) | 25 ± 15 | 21 ± 7 | 20 ± 4 | N.S. |
| Urea nitrogen (mg/dL) | 14 ± 2 | 14 ± 2 | 14 ± 2 | N.S. |
| Creatinine (mg/dL) | 0.2 ± 0.1 | 0.2 | 0.3 ± 0.1 | N.S. |
| AST (U/L) | 108 ± 24 | 68 ± 2 | 72 ± 5 | N.S. |
| ALT (U/L) | 31 ± 2 | 24 ± 3* | 25 ± 1* | *p<0.05 |
| ALP (U/L) | 583 ± 135 | 470 ± 112 | 540 ± 64 | N.S. |
| CK (U/L) | 162 ± 31 | 170 ± 22 | 196 ± 12 | N.S. |
| Sodium (mmol/L) | 143 ± 1 | 143 | 143 ± 1 | N.S. |
| Pottasium (mmol/L) | 3.4 ± 0.2 | 3.6 ± 0.2 | 3.6 ± 0.2 | N.S. |
| Chloride (mmol/L) | 104 ± 1 | 103 ± 1 | 105 ± 1 | N.S. |
| Calcium (mmol/L) | 10.2 ± 0.4 | 10.1 ± 0.1 | 9.9 ± 0.3 | N.S. |

As seen from Table 2, no particular toxicity due to intrarectal administration of PFD was detected.

Under a hypoxic environment, oxygen partial pressure in the inferior vena cava was significantly higher in the ILV group than in the control group (120 min, p = 0.037), and the pressure difference between the two groups was 9.40 ± 3.65 mmHg (Figure 3C). Likewise, oxygen partial pressure in the left ventricle of the heart under a hypoxic environment was significantly higher in the ILV group than in the control group 60 minutes after ILV (Figure 3D), and after 120 minutes a pressure difference between the two groups (23.8 ± 3.11 mmHg) was observed in a more significant manner (p = 0.020).

Next, the ventricular oxygen partial pressure of the heart of that model after PFC treatment was compared with that of a mucosal-abrasion-involving hypoxic model. The result showed that the ventricular oxygen partial pressure of the heart under a hypoxic environment in the IGV group was comparable to that in the ILV group (IGV group; PaO₂ 63.3 ± 6.95 mmHg, ILV group: PaO₂ 63.8 ± 5.59 mmHg, p = 0.80). These data suggest that the hypoxic mouse model can sufficiently recover oxygen concentration with administration of O₂-containing PFC to the intestinal tract, and does not need mucosal abrasion for the intestinal tract (distal intestine).

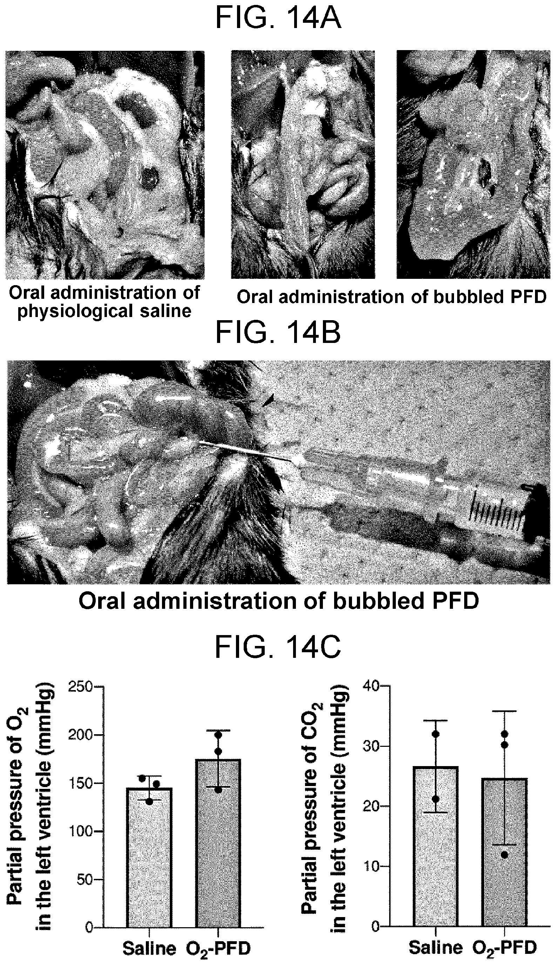

PFD subjected to oxygen bubbling was administered to mice through oral administration, and the effects on blood oxygen partial pressure and carbon dioxide partial pressure were examined. As a negative control, a mouse group with oral administration of physiological saline (bubbled-PFD oral administration group; n = 3) was used. For a mouse group with oral administration of PFD subjected to oxygen bubbling (bubbled PDF) (n = 3), a volume of oxygen-containing PFD (250 µL/mouse) was administered to the stomach by using a probe, and the intestinal tract was observed to find that transparent liquid was contained in the intestinal tract, but only solid matters and air were contained in the stomach (Figure 14A). In the negative control group, in contrast to that, there were smaller amounts of contents in the intestinal tract than in the bubbled-PFD oral administration group (Figure 14A). An attempt was made to recover contents by inserting a syringe into the intestinal tract. In the attempt, transparent liquid suspected to be PFD was successfully recovered in the syringe for the bubbled-PFD oral administration group (Figure 14B). For the negative control group, by contrast, such liquid was not recovered, and nothing recovered was left in the syringe. These results indicated that orally administered PDF passed through the stomach and arrived at the intestine. It was also confirmed that the PDF that had arrived at the intestine did not return to the stomach. One hour after administration, tracheostomy was performed, and ventilation was performed with room air under management with a ventilator. Thoracotomy was performed and blood was collected from the heart, and the oxygen partial pressure and carbon dioxide partial pressure in the blood obtained were measured by iSTAT. The results were as shown in Figure 14C. As seen from Figure 14C, the bubbled-PFD oral administration group was found to have a tendency to exhibit increased blood oxygen partial pressure as compared with the negative control group, and a tendency to exhibit decreased blood carbon dioxide partial pressure. These results demonstrated that PFC dissolving oxygen therein is capable of increasing blood oxygen partial pressure and decreasing carbon dioxide partial pressure through any of intrarectal administration and oral administration.

### [Discussion]

The present invention has verified the proof-of-principle approach to demonstrate that intestinal ventilation ameliorates type I respiratory failure in mice. Type I respiratory failure is the most common form of respiratory failure, and characterized by an arterial oxygen partial pressure lower than 60 mmHg in combination with normal or low carbon dioxide partial pressure due to the failure of gas change functions. Inhalation of hypoxic gas causes hyperpnea and decreased carbon dioxide partial pressure. The respiratory system depends on the following three factors: 1. concentration or partial pressure of oxygen, 2. dissolution rate of oxygen passing through broad surfaces of alveoli containing fat-soluble surfactant, and 3. efficient transport of oxygen passing through thin interstitial tissue to blood vessels. Our experimental system ameliorated respiratory failure by applying the intestinal respiratory mechanism with both IGV and ILV to enhance oxygen partial pressure and in turn increase the systemic transport of oxygen. In addition, the present invention demonstrated that intestinal ventilation decreases blood carbon dioxide partial pressure. This provides a therapeutic strategy against type II respiratory failure involving increased blood carbon dioxide partial pressure. Thus, according to the present invention, PFC can be utilized as a gas-exchange platform that is capable of supplying oxygen into blood and absorbing carbon dioxide from blood.

Intestinal respiration has been reported for loaches, corydorases, and sea cucumbers^{7,20}, whereas no examination has been made for mammalian systems. Histopathological findings have shown that the posterior intestine of loaches has a thin mucosal epithelium, and is rich in blood vessels. Moreover, significant increase is found for genes associated with angiogenesis in the posterior intestine of loaches^{11,18,20-24}. The mechanical mucosal abrasion for our mouse models enabled, as found in those findings, both thinning of the epithelium and induction of angiogenic factors observed in the acute phase. These physiological features and molecular signatures are highly probably important factors for the efficient oxygenation observed in our model system.

PFC causes side effects including increased blood pressure and organ dysfunction when being directly administered to a blood vessel or airway^{13,14,16,25}. However, we employed PFC as a liquid-based oxygen carrier to develop an intestinal ventilation system that does not need mechanical mucosal scraping and plays a more vital role in clinical situations. On being introduced to an intestinal tract region highly enriched with blood vessels (e.g., the rectum, which has a rectal venous plexus), PFC immediately permeated tissue and migrated to microvessels. As expected, the ratio of arterial oxygen partial pressure between the control group and the treated group was approximately 1.6 (40.0 ± 2.94 mmHg and 63.8 ± 5.59 mmHg, respectively), and type I respiratory failure was ameliorated. If application to humans is intended, that increase in the ratio is enough for treating patients having severe respiratory failure. Suppose that the total volume of the body fluid of a human is 1,000 times that of a mouse, l to 2 L or more of liquid PFC should be needed per day to ameliorate serious respiratory failure in a group with acute respiratory distress syndrome or the like. For example, it is also contemplated to administer PFC supplemented with oxygen in a glycerin enema into the intestinal tract (in particular, into the large intestine or into the rectum) every 6 to 8 hours in divided doses. Even in the atmosphere, PFC dissolves oxygen therein by absorbing oxygen from the atmosphere. Accordingly, PFC maintained in the atmosphere could be administered to the rectum. PFC that has been provided with an increased amount of oxygen dissolved therein by oxygen bubbling may be administered. Furthermore, a hybrid mode of administration with IGV and ILV can also serve as an effective oxygenation technique. Specifically, administration of oxygen gas after administration of liquid PFC allows the oxygen saturation of the PFC remaining in the intestinal tract (in particular, in the large intestine or in the rectum) to be repeatedly enhanced, and addition of treatment to repeat such a cycle is expected to enable sustainable improvement of oxygen supply from PFC to blood. Moreover, the potential of oxygen-containing PFC to supply oxygen through oral administration and the potential thereof to discharge carbon dioxide via the stomach were demonstrated. This will open the way to more convenient oxygen supply.

### References

1. Tobin M, Manthous C. Mechanical Ventilation. Am J Respir Crit Care Med 2017; 196: P3-P4.2. Pham T, Brochard LJ, Slutsky AS. Mechanical Ventilation: State of the Art. Mayo Clin Proc 2017; 92: 1382-400.
3. Patel B, Chatterjee S, Davignon S, Herlihy JP. Extracorporeal membrane oxygenation as rescue therapy for severe hypoxemic respiratory failure. J Thorac Dis 2019; 11: S1688-S97.
4. Henry BM. COVID-19, ECMO, and lymphopenia: a word of caution. Lancet Respir Med 2020; 8: e24.
5. Yang X, Yu Y, Xu J, et al. Clinical course and outcomes of critically ill patients with SARS-CoV-2 pneumonia in Wuhan, China: a single-centered, retrospective, observational study. Lancet Respir Med 2020.
6. Nakazawa MS, Keith B, Simon MC. Oxygen availability and metabolic adaptations. Nat Rev Cancer 2016; 16: 663-73.
7. Plaul SE, Barbeito CG, Diaz AO. Histochemical differences along the intestine of Corydoras paleatus (Siluriformes: Callichthyidae). Rev Biol Trop 2016; 64: 327-40.
8. Bickford D, Iskandar D, Barlian A. A lungless frog discovered on Borneo. Curr Biol 2008; 18: R374-5.
9. Woods HA, Lane SJ, Shishido C, Tobalske BW, Arango CP, Moran AL. Respiratory gut peristalsis by sea spiders. Curr Biol 2017; 27: R638-R9.
10. Park TJ, Reznick J, Peterson BL, et al. Fructose-driven glycolysis supports anoxia resistance in the naked mole-rat. Science 2017; 356: 307-11.
11. Huo D, Sun L, Ru X, et al. Impact of hypoxia stress on the physiological responses of sea cucumber Apostichopus japonicus: respiration, digestion, immunity and oxidative damage. PeerJ 2018; 6 :e4651.
12. Caridi-Scheible ME, Blum JM. Use of Perfluorodecalin for Bronchoalveolar Lavage in Case of Severe Pulmonary Hemorrhage and Extracorporeal Membrane Oxygenation: A Case Report and Review of the Literature. A A Case Rep 2016; 7: 215-8.
13. Ferrari RS, Thomaz L, Simoneti LEL, Ulbrich JM, Andrade CF. Effect of vaporized perfluorocarbon on oxidative stress during the cold ischemia phase of lung graft preservation. J Bras Pneumol 2019; 45: e20170288.
14. Forgiarini Junior LA, Holand AR, Forgiarini LF, et al. Endobronchial perfluorocarbon reduces inflammatory activity before and after lung transplantation in an animal experimental model. Mediators Inflamm 2013; 2013: 193484.
15. Riess JG. Understanding the fundamentals of perfluorocarbons and perfluorocarbon emulsions relevant to in vivo oxygen delivery. Artif Cells Blood Substit Immobil Biotechnol 2005; 33: 47-63.
16. Yu Q, Liu K, Su L, Xia X, Xu X. Perfluorocarbon liquid: its application in vitreoretinal surgery and related ocular inflammation. Biomed Res Int 2014; 2014: 250323.
17. Aguilera KY, Brekken RA. Hypoxia Studies with Pimonidazole in vivo. Bio Protoc 2014; 4.
18. Luo W, Cao X, Xu X, Huang S, Liu C, Tomljanovic T. Developmental transcriptome analysis and identification of genes involved in formation of intestinal air-breathing function of Dojo loach, Misgurnus anguillicaudatus. Sci Rep 2016; 6: 31845.
19. Kuznetsova IN. Stability of perfluorocarbon emulsions and their compatibility with blood serum. Artif Cells Blood Substit Immobil Biotechnol 1998; 26: 181-9.
20. Huo D, Sun L, Li X, et al. Differential Expression of miRNAs in the Respiratory Tree of the Sea Cucumber Apostichopus japonicus Under Hypoxia Stress. G3 (Bethesda) 2017; 7: 3681-92.
21. Huang S, Cao X, Tian X. Transcriptomic Analysis of Compromise Between Air-Breathing and Nutrient Uptake of Posterior Intestine in Loach (Misgurnus anguillicaudatus), an Air-Breathing Fish. Mar Biotechnol (NY) 2016; 18: 521-33.
22. Fish JE, Cantu Gutierrez M, Dang LT, et al. Dynamic regulation of VEGF-inducible genes by an ERK/ERG/p300 transcriptional network. Development 2017; 144: 2428-44.
23. Wang Y, Nakayama M, Pitulescu ME, et al. Ephrin-B2 controls VEGF-induced angiogenesis and lymphangiogenesis. Nature 2010; 465: 483-6.
24. Wilson JM, Moreira-Silva J, Delgado IL, et al. Mechanisms of transepithelial ammonia excretion and luminal alkalinization in the gut of an intestinal air-breathing fish, Misgurnus anguilliacaudatus. J Exp Biol 2013; 216: 623-32.
25. Chambers DC, Cherikh WS, Goldfarb SB, et al. The International Thoracic Organ Transplant Registry of the International Society for Heart and Lung Transplantation: Thirty-fifth adult lung and heart-lung transplant report-2018; Focus theme: Multiorgan Transplantation. J Heart Lung Transplant 2018; 37: 1169-83.

## Claims

1. A pharmaceutical composition for oral administration, nasogastric administration, trans-fistula gastric administration, or administration into a large intestine, the pharmaceutical composition comprising a perfluorocarbon dissolving oxygen therein.

2. The pharmaceutical composition according to claim 1, for use in treating hypoxemia.

3. The pharmaceutical composition according to claim 1 or 2, wherein the pharmaceutical composition is administered to a subject having respiratory failure.

4. The pharmaceutical composition according to any one of claims 1 to 3, for use in supplying oxygen to blood of a subject.

5. An administration device for administration into a large intestine, the administration device comprising the pharmaceutical composition according to any one of claims 1 to 4.

6. A pharmaceutical composition in a form of gas, comprising oxygen gas, for oral administration, nasogastric administration, trans-fistula gastric administration, or administration into a large intestine, wherein
the large intestine is a large intestine subjected to mucosal removal or a large intestine coated with a perfluorocarbon.

7. A composition in a form of gas, comprising oxygen gas, wherein the composition is dissolved in a perfluorocarbon before administration and administered through oral administration, nasogastric administration, trans-fistula gastric administration, or administration into a large intestine.

8. The composition according to any one of claims 1 to 4, wherein the composition is mixed with oxygen gas before administration and administered through oral administration, nasogastric administration, trans-fistula gastric administration, or administration into a large intestine.

9. A composition in a form of gas, comprising oxygen gas, for use in administration into a large intestine to a subject having a large intestine subjected to mucosal removal or a large intestine coated with a perfluorocarbon.

10. The composition according to any one of claims 1 to 4 and 6 to 9 for intrarectal administration.

11. The composition according to any one of claims 1 to 4 and 6 to 9 for oral administration, nasogastric administration, or trans-fistula gastric administration.

12. The composition according to any one of claims 1 to 4 and 6 to 11 for use in decreasing blood carbon dioxide partial pressure of a subject.

13. A kit for pre-use preparation of a composition for administration into a large intestine, the kit comprising: a composition in a form of gas, comprising oxygen gas; and a composition comprising a perfluorocarbon.

14. The kit for pre-use preparation according to claim 13 for use in increasing blood oxygen partial pressure of a subject.

15. The kit for pre-use preparation according to claim 13 or 14 for use in decreasing blood carbon dioxide partial pressure of a subject.

16. An administration device for administration into a large intestine, the device comprising the composition according to any one of claims 1 to 4 and 6 to 12.

17. An administration controller for oral administration, nasogastric administration, trans-fistula gastric administration, or administration into a large intestine of a perfluorocarbon dissolving oxygen therein or oxygen, the administration controller comprising: a delivering unit configured to deliver the perfluorocarbon dissolving oxygen therein or oxygen to a tube; and a controlling unit configured to control a rate of delivery from the delivering unit on the basis of blood oxygen saturation of a subject and intraintestinal pressure of a large intestine of the subject.

18. The administration controller according to claim 17, further comprising a receiving unit configured to receive information on oxygen saturation from a blood oxygen monitor and intraintestinal pressure of a large intestine.

19. The administration controller according to claim 17 or 18, comprising:
a controlling unit configured to control a rate of delivery from the delivering unit on the basis of blood oxygen saturation of the subject, wherein
(A) the controlling unit refers to information on oxygen saturation received by a receiving unit, and if the oxygen saturation has reached lower than a predetermined value or a predetermined value or lower, the controlling unit sends a signal to increase the rate of delivery to the delivering unit; and/or
(B) the controlling unit refers to information on oxygen saturation received by a receiving unit, and if the oxygen saturation has reached a predetermined value or higher or higher than a predetermined value, the controlling unit sends a signal to decrease the rate of delivery to the delivering unit.

20. The administration controller according to any one of claims 17 to 19, further comprising:
a controlling unit configured to control a rate of delivery from the delivering unit on the basis of intraintestinal pressure of a large intestine of the subject, wherein
(C) the controlling unit refers to information on intraintestinal pressure of a large intestine received by a receiving unit, and if the intraintestinal pressure has reached lower than a predetermined value or a predetermined value or lower or if the intraintestinal pressure has increased during liquid delivery, the controlling unit stops sending a signal to increase the rate of delivery to the delivering unit, or sends a signal to decrease the rate of delivery to the delivering unit.

21. A method for administering oxygen to a subject, the method comprising:
administering a pharmaceutical composition comprising a perfluorocarbon dissolving oxygen therein to the subject through oral administration, nasogastric administration, trans-fistula gastric administration, or administration into a large intestine.

22. The method according to claim 21, comprising:
administering a perfluorocarbon dissolving oxygen therein to the subject through oral administration, nasogastric administration, trans-fistula gastric administration, or administration into a large intestine with dose control using an administration controller, wherein
the administration controller is an administration controller for administering a perfluorocarbon dissolving oxygen therein or oxygen, and comprises: a delivering unit configured to deliver the perfluorocarbon dissolving oxygen therein or oxygen to a tube; and a controlling unit configured to control a rate of delivery from the delivering unit on the basis of blood oxygen saturation of the subject and/or intraintestinal pressure of a large intestine of the subject.

23. The method according to claim 22 or 23, for a rectum in a large intestine.

24. The composition according to any one of claims 1 to 4 and 6 to 12, being a pharmaceutical composition for administering a perfluorocarbon dissolving oxygen therein to the subject through oral administration, nasogastric administration, trans-fistula gastric administration, or administration into a large intestine with dose control using an administration controller, wherein
the administration controller is an administration controller for administering a perfluorocarbon dissolving oxygen therein or oxygen, and comprises: a delivering unit configured to deliver the perfluorocarbon dissolving oxygen therein or oxygen to a tube; and a controlling unit configured to control a rate of delivery from the delivering unit on the basis of blood oxygen saturation of the subject and/or intraintestinal pressure of a large intestine of the subject.
